(19) Europäisches Patentamt
European Patent Office
Office européen des brevets

(11) EP 0 541 081 B1

(12) # EUROPÄISCHE PATENTSCHRIFT

(45) Veröffentlichungstag und Bekanntmachung des Hinweises auf die Patenterteilung:
**10.04.2002 Patentblatt 2002/15**

(21) Anmeldenummer: **92118951.0**

(22) Anmeldetag: **05.11.1992**

(51) Int Cl.[7]: **C07D 285/12**, C09K 19/34, C09K 19/30, C07D 239/26, C07D 319/06, C07D 213/30, C07C 69/92, C07D 401/04

(54) **Verbindungen mit nur einer Seitenkette zur Verwendung in Flüssigkristallmischungen**

Compounds with only a side chain for use in liquid crystal mixtures

Composés avec seulement une chaîne latéralle pour utilisation dans des mélanges de cristaux liquides

(84) Benannte Vertragsstaaten:
**AT BE CH DE ES FR GB IT LI NL SE**

(30) Priorität: **07.11.1991 DE 4136627**

(43) Veröffentlichungstag der Anmeldung:
**12.05.1993 Patentblatt 1993/19**

(73) Patentinhaber: **Aventis Research & Technologies GmbH & Co. KG**
**65926 Frankfurt am Main (DE)**

(72) Erfinder:
- **Illian, Gerhard, Dr.**
  **Nerimaku Tokyo 176 (JP)**
- **Kaltbeitzel, Anke, Dr.**
  **W-6090 Rüsselsheim (DE)**
- **Wingen, Rainer, Dr.**
  **W-6234 Hattersheim/M. (DE)**
- **Schlosser, Hubert, Dr.**
  **W-6246 Glashütten/Ts. (DE)**

(74) Vertreter: **Isenbruck, Günter, Dr. et al**
**Patent- und Rechtsanwälte,**
**Bardehle-Pagenberg-Dost-Altenburg-Geissler-Isenbruck**
**Theodor-Heuss-Anlage 12**
**68165 Mannheim (DE)**

(56) Entgegenhaltungen:

| | |
|---|---|
| **EP-A- 0 022 882** | **EP-A- 0 087 679** |
| **EP-A- 0 090 671** | **EP-A- 0 097 033** |
| **EP-A- 0 154 840** | **EP-A- 0 196 070** |
| **EP-A- 0 336 619** | **EP-A- 0 354 655** |
| **EP-A- 0 381 149** | **WO-A-88/02390** |
| **DD-A- 117 014** | **DE-A- 2 933 544** |

- **JOURNAL OF ORGANIC CHEMISTRY Bd. 38, Nr. 18 , 1973 , EASTON US Seite 31603164 J. P. SCHROEDER 'Liquid Crystals. IV. Effects of Terminal Substituents on the Nematic Mesomorphism of p-Phenylene Dibenzoates'**
- **MOLECULAR CRYSTALS AND LIQUID CRYSTALS (INC. NONLINEAR OPTICS ) Bd. 173 , 1989 , READING GB Seiten 121 - 140 J. W. BROWN 'Some Three-Ring Esters Containing a Five-Membered Heteroaromatic Ring.'**
- **MOLECULAR CRYSTALS AND LIQUID CRYSTALS (INC. NONLINEAR OPTICS ) Bd. 62, Nr. 1/2 , 1980 , READING GB Seiten 103 - 114 D. J. BYRON 'Properties of the Liquid Crystals Formed by Certain 4-(2'- Pyridyl)phenyl and 4-(4'- Pyridyl)phenyl 4''-n-Alkoxybenzoates'**
- **JOURNAL FÜR PRAKTISCHE CHEMIE Bd. 315, Heft 6, 1973, LEIPZIG DD, S. 1113-20 H. ZASCHKE ' Kristalin-flüssige 2,5-Diphenyl-pyrimidine mit nur einer Flügelgruppe '**
- **JOURNAL FÜR PRAKTISCHE CHEMIE Bd. 321, Heft 4, 1979, LEIPZIG DD, S. 643-654 K. DöLLING ' Kristallin-flüssige Thiazole '**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 541 081 B1

## Beschreibung

Flüssigkristalle haben insbesondere im letzten Jahrzehnt Eingang in verschiedene technische Gebiete gefunden, in denen elektrooptische und Anzeigevorrichtungs-Eigenschaften gefragt sind (z. B. in Uhren-, Taschenrechner- und Schreibmaschinenanzeigen). Diese Anzeigevorrichtungen beruhen auf den dielektrischen Ausrichtungseffekten in den nematischen, cholesterischen und/oder smektischen Phasen der flüssigkristallinen Verbindungen, wobei - verursacht durch die dielektrische Anisotropie - die molekulare Längsachse der Verbindungen eine bevorzugte Ausrichtung in einem angelegten elektrischen Feld einnimmt. Die üblichen Schaltzeiten bei diesen Anzeigevorrichtungen sind für viele andere potentielle Anwendungsgebiete von Flüssigkristallen zu lang. Dieser Nachteil macht sich insbesondere dann bemerkbar, wenn eine große Anzahl von Bildpunkten angesteuert werden muß. Die Herstellungskosten von Geräten, die größere Bildschirmflächen enthalten, sind dann im allgemeinen zu hoch.

Neben den nematischen und cholesterischen Flüssigkristallen haben seit einigen wenigen Jahren in zunehmendem Maße auch optisch aktive smektische Flüssigkristall-Phasen an Bedeutung gewonnen.

Clark und Lagerwall konnten zeigen, daß die Verwendung ferroelektrischer Flüssigkristallsysteme in sehr dünnen Zellen zu elektrooptischen Schalt- oder Anzeigeelementen führt, die im Vergleich zu den herkömmlichen TN ("twisted nematic")-Zellen um bis zu einem Faktor 1000 schnellere Schaltzeiten haben (vgl. z.B. Lagerwall et al. "Ferroelectric Liquid Crystals for Displays", SID Symposium, October Meeting 1985, San Diego, Ca., USA). Aufgrund dieser und anderer günstiger Eigenschaften, z.B. der bistabilen Schaltmöglichkeit und des nahezu blickwinkelunabhängigen Kontrasts, sind FLCs grundsätzlich für die obengenannten Anwendungsgebiete, z.B. über eine Matrixansteuerung, gut geeignet. Auch auf dem Gebiet der räumlichen Lichtmodulatoren (vgl. z. B. U. Efron in "Spatial Light Modulators and Applications", SPIE Vol. 1150, S. 46 ff) sind ferroelektrische Flüssigkristalle wegen ihres hohen Kontrasts und Geschwindigkeit besonders geeignet. Die Geschwindigkeit ferroelektrischer Flüssigkristallmischungen reicht im allgemeinen aber noch nicht aus, um z. B. hochauflösende, schnelle Anzeigeelemente zu betreiben. Daher ist es wünschenswert, Komponenten aufzufinden, die die Schaltgeschwindigkeit flüssigkristalliner Mischungen steigern. Gegenstand der Erfindung sind deshalb Komponenten, die die Schaltzeit von Flüssigkristallmischungen herabsetzen.

Flüssigkristalle mit nur einer Seitenkette wurden wenig untersucht. Beispiele finden sich in: D. Demus, H. Zaschke, "Flüssigkristalle in Tabellen", Bd. 2, 1. Aufl., VEB Deutscher Verlag für Grundstoffindustrie, Leipzig 1984, S. 360. Da die Phaseneigenschaften dieser Stoffe im Vergleich zu mesogenen Verbindungen mit zwei Seitenketten ungünstig erschienen, sind sie in Mischungen bisher nicht eingesetzt worden.

Es wurde nun überraschend gefunden, daß Verbindungen gemäß Formel I mit nur einer Seitenkette die Schaltgeschwindigkeit von Flüssigkristallmischungen erheblich steigern.

Gegenstand der Erfindung ist die Verwendung von Verbindungen der allgemeinen Formel I

$$R^1(-A^1)_a(-M^1)_b(-A^2)_c(-M^2)_d(-A^3)_e(-M^3)_f(-A^4)_g\text{-H} \qquad (I)$$

in der die Symbole und Indices folgende Bedeutung haben:

$R^1$ ist ein geradkettiger oder verzweigter Alkylrest mit 1 bis 22 C-Atomen (mit oder ohne asymmetrisches C-Atom), wobei auch eine oder zwei nicht benachbarte $-CH_2$-Gruppen durch -O-, -S-, -CO-, -CO-O-, -O-CO-, -CO-S-, -S-CO-, -O-CO-O-, -CH=CH-, -C≡C-, oder $-Si(CH_3)_2$- ersetzt sein können und wobei auch ein oder mehrere H-Atome des Alkylrestes durch F, Cl, Br oder CN substituiert sein können, oder ist eine der nachfolgenden chiralen Gruppen:

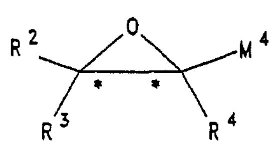
,
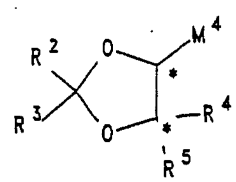
,

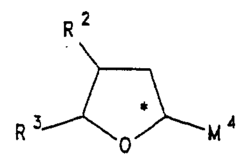 , 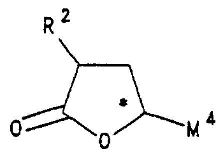 ,

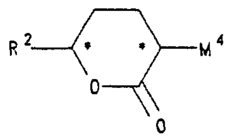 , 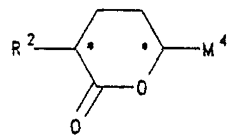 ,

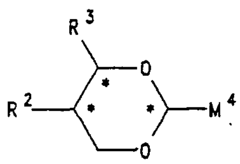 , 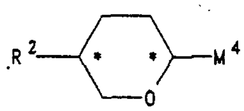 ,

H H H H
| | | |
$R^2$-*C-CO-O-, $R^2$-*C-CO-O-, $R^2$-*C-$CH_2$-O-, $R^2$-*C-$CH_2$-O-,
| | | |
Cl F Cl F

H H
| |
$R^2$-*C-$CH_2$CO-O-, $R^2$-*C-$CH_2CH_2$-O-,
| |
Cl Cl

$CH_3$ $CH_3$
| |
$R^2$-O-*C-CO-O-, $R^2$-O-CO-*C-O-,
| |
H H

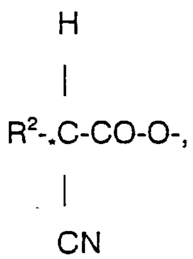 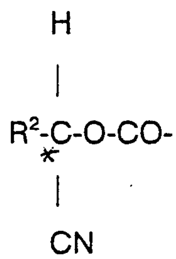

$R^2$, $R^3$, $R^4$, $R^5$ sind unabhängig voneinander H oder ein geradkettiger oder verzweigter Alkylrest mit 1 bis 22 C-Atomen, wobei auch eine oder zwei nicht benachbarte $-CH_2$-Gruppen durch -O-, -S-, -CO-, -CO-O-, -O-CO-, -CO-S-, -S-CO-, -O-CO-O-, -CH=CH-, -C≡C-, oder -Si(CH3)2- ersetzt sein können, oder $R^2$ und $R^3$ können zusammen auch $-(CH_2)_4-$ oder $-(CH_2)_5-$ sein, wenn sie als Substituenten an ein Dioxolan-System gebunden sind;

$A^1$, $A^2$, $A^3$, $A^4$ sind gleich oder verschieden 1,4-Phenylen, wobei ein oder zwei H-Atome durch F, Cl und/oder CN ersetzt sein können, Pyrazin-2,5-diyl, Pyridazin-3,6-diyl, Pyridin-2,5-diyl, Pyrimidin-2,5-diyl, bei dem ein oder zwei H-Atome durch F ersetzt sein können, trans-1,4-Cyclohexylen, (1,3,4)-Thiadiazol-2,5-diyl, 1,3-Dioxan-2,5-diyl, 1,3-Dithian-2,5-diyl, 1,3-Thiazol-2,4-diyl, 1,3-Thiazol-2,5-diyl, Thiophen-2,4-diyl, Thiophen-2,5-diyl, Piperazin-1,4-diyl, Piperazin-2,5-diyl, Naphthalin-2,6-diyl, Bicyclo[2.2.2]octan-1,4-diyl, 1,3-Dioxaborinan-2,5-diyl oder trans-Dekalin-2,6-diyl;

$M^1$, $M^2$, $M^3$ sind gleich oder verschieden, -O-, -S-, -CO-, -CO-O-, -O-CO-, -CO-S-, -S-CO-, -O-CO-O-, $-CH_2-O-$, $-O-CH_2-$, $-CH_2CH_2-$, -CH=CH- oder -C≡C-;

$M^4$ ist $-CH_2-O-$, $-O-CH_2-$, -CO-O-, -O-CO- oder eine Einfachbindung;

b, c, d, e, f, sind null oder eins, a und g sind 1,

* ist ein chirales Zentrum;

in ferroelektrischen Flüssigkristallmischungen.

Bevorzugt sind Verbindungen der allgemeinen Formel (I), in der die Symbole und Indices folgende Bedeutung haben:

$R^1$ ist ein geradkettiger oder verzweigter Alkylrest mit 1 bis 22 C-Atome (mit oder ohne asymmetrisches C-Atom), wobei auch eine oder zwei nicht benachbarte $-CH_2$-Gruppen durch -O-, -S-, -CO-, -CO-O-, -O-CO-, -O-CO-O-, -CH=CH-, -C≡C- oder
-Si(CH3)2)- ersetzt sein können, oder eine der nachfolgenden chiralen Gruppen:

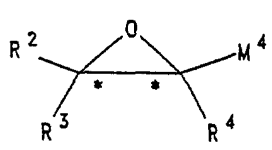 , 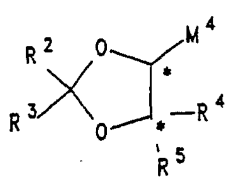 ,

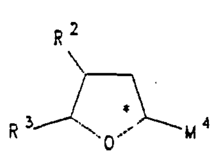 , 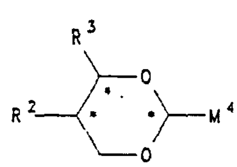 ,

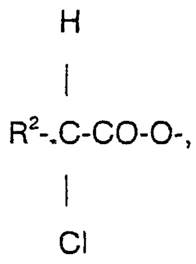 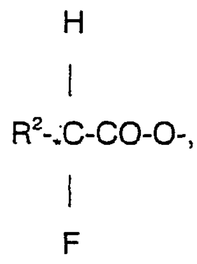 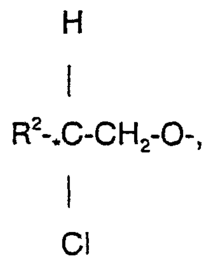 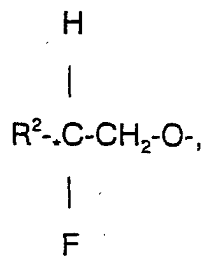

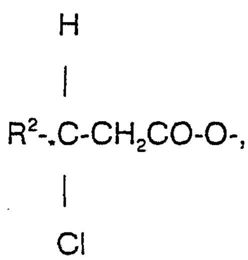 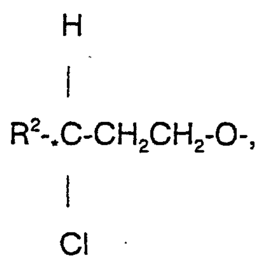

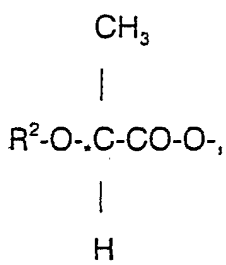 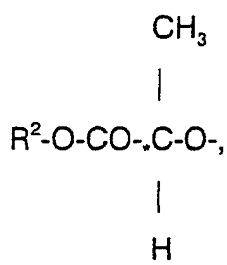

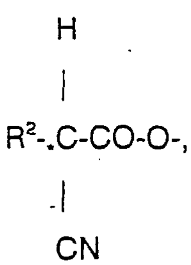 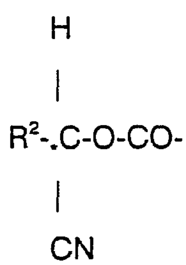

$R^2$, $R^3$, $R^4$, $R^5$ sind unabhängig voneinander H oder ein geradkettiger oder verzweigter Alkylrest mit 1 bis 22 C-Atomen, wobei auch eine oder zwei nicht benachbarte $-CH_2$-Gruppen durch -O-, -S-, -CO-, -CO-O-, -O-CO-, -CO-S-, -S-CO-, -O-CO-O-, -CH=CH-, -C≡C- oder -Si(CH3)2- ersetzt sein können, oder $R^2$ und $R^3$ können zusammen auch $-(CH_2)_4-$ oder $-(CH_2)_5-$ sein, wenn sie als Substituenten an ein Dioxolan-System gebunden sind;

$A^1$, $A^2$, $A^3$, $A^4$ sind gleich oder verschieden 1,4-Phenylen, Pyrazin-2,5-diyl, Pyridazin-3,6-diyl, Pyridin-2,5-diyl, Pyrimidin-2,5-diyl, trans-1,4-Cyclohexylen, (1,3,4)-Thiadiazol-2,5-diyl, 1,3-Dioxan-2,5-diyl, Naphthalin-2,6-diyl oder Bicyclo[2.2.2]octan-1,4-diyl;

$M^1$, $M^2$, $M^3$ sind gleich oder verschieden -O-, -CO-, -CO-O-, -O-CO-, $-CH_2-O-$, $-O-CH_2-$, $-CH_2CH_2-$, -CH=CH- oder -C≡C-;

$M^4$ ist $-CH_2-O-$, $-O-CH_2-$, -CO-O-, -O-CO- oder eine Einfachbindung.

Besonders bevorzugt sind Verbindungen der allgemeinen Formel (I), in der die Symbole und Indices folgende Bedeutung haben:

$R^1$ ist ein geradkettiger oder verzweigter Alkylrest mit 1 bis 22 C-Atomen (mit oder ohne asymmetrisches C-Atom), wobei auch eine oder zwei nicht benachbarte $-CH_2$-Gruppen durch -O-, -CO-, -CO-O-, -O-CO-, -CH=CH-, -C≡C- oder $-Si(CH_3)_2-$ ersetzt sein können, oder eine der nachfolgenden chiralen Gruppen:

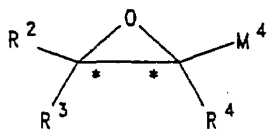
,
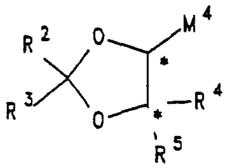
,

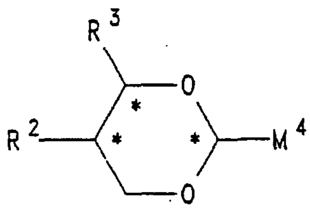
,

H
|
$R^2$-*C-CO-O-,
|
Cl

H
|
$R^2$-*C-CO-O-,
|
F

$CH_3$
|
$R^2$-O-*C-CO-O-,
|
H

$CH_3$
|
$R^2$-O-CO-*C-O-,
|
H

H
|
$R^2$-*C-CO-O-,
|
CN

$R^2$, $R^3$, $R^4$, $R^5$ sind unabhängig voneinander H oder ein geradkettiger oder verzweigter Alkylrest mit 1 bis 22 C-Atomen, wobei auch eine oder zwei nicht benachbarte $-CH_2$-Gruppen durch -O-, -S-, -CO-, -CO-O, -O-CO-, -CO-S-, -S-CO-, -O-CO-O-, -CH=CH-, -C≡C- oder $-Si(CH_3)_2-$ ersetzt sein können, oder $R^2$ und $R^3$ können zusammen auch $-(CH_2)_4-$ oder $-(CH_2)_5-$ sein, wenn sie als Substituenten an ein Dioxolan-System gebunden sind;

$A^1$, $A^2$, $A^3$, $A^4$ sind gleich oder verschieden 1,4-Phenylen, Pyrazin-2,5-diyl, Pyridin-2,5-diyl, Pyrimidin-2,5-diyl, trans-1,4-Cyclohexylen, (1,3,4)-Thiadiazol-2,5-diyl, Naphthalin-2,6-diyl oder 1,3-Dioxan-2,5-diyl
$M^1$, $M^2$, $M^3$ sind gleich oder verschieden, -O-, -CO-O-, -O-CO-, $-CH_2-O-$, $-O-CH_2-$, $-CH_2-CH_2-$, -CH=CH- oder -C≡C-;
$M^4$ ist $-CH_2-O-$, $-O-CH_2-$, -CO-O-, -O-CO- oder eine Einfachbindung.

Es sind insbesondere Verbindungen der allgemeinen Formel (I) bevorzugt, in der $R^1$ ein Alkylrest mit 1 bis 22 C-Atomen ist, wobei eine $-CH_2$-Gruppe durch -O-, -CH=CH- oder $-Si(CH_3)_2-$ ersetzt sein kann oder die chirale Gruppe

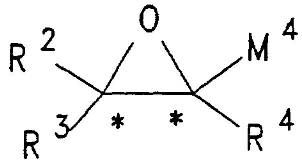

und die Gruppierung $(-A^1)_a(-M^1)_b(-A^2)_c(-M^2)_d(-A^3)_e(-M^3)_f(-A^4)_g$ folgende Bedeutung hat:

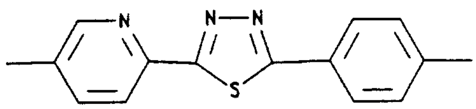 ,

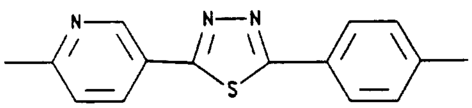 , 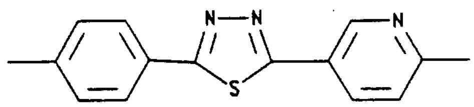 ,

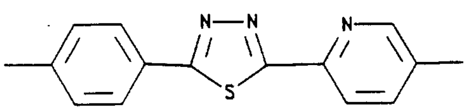 , 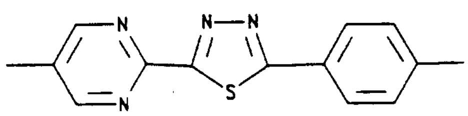 ,

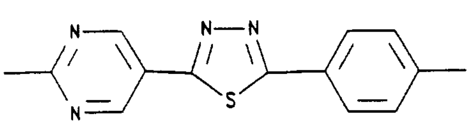 , 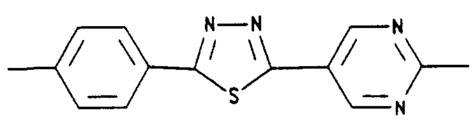 ,

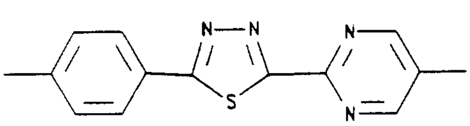 , 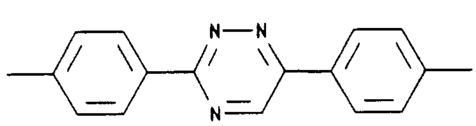 ,

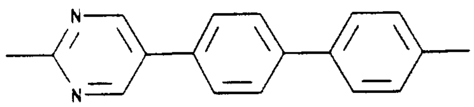 , 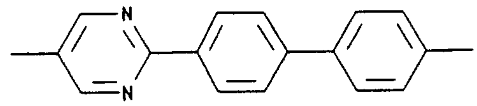 ,

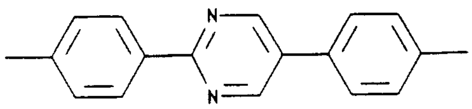 , 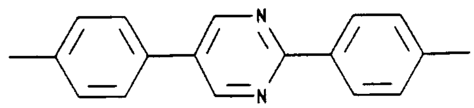 ,

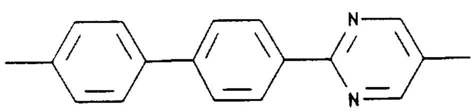 , 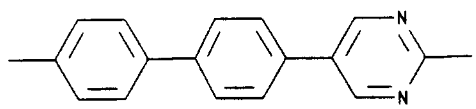 ,

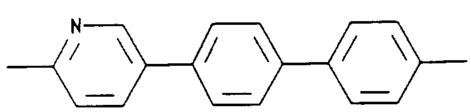 , 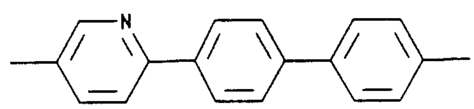 ,

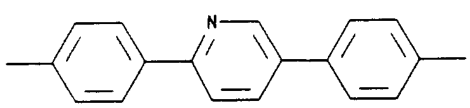 , 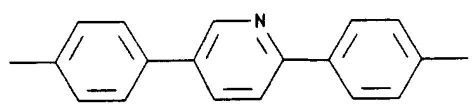 ,

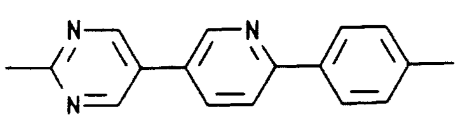 , 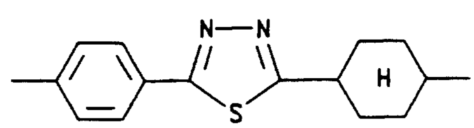

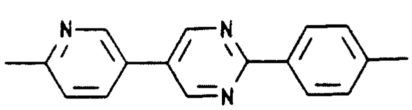 , 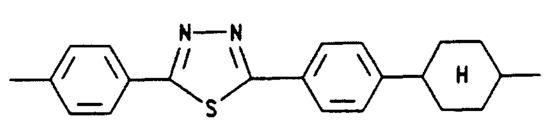

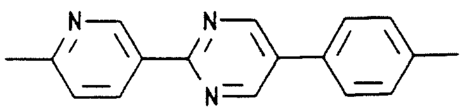 , 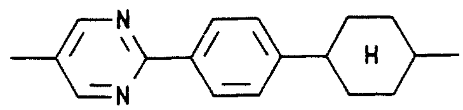

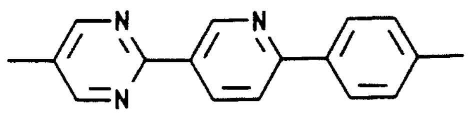

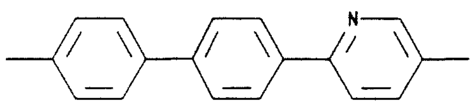 , 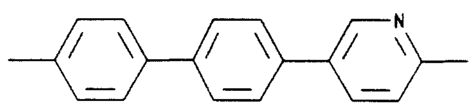 ,

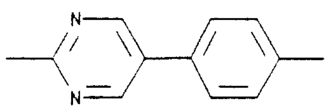 , 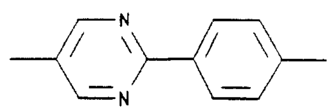 ,

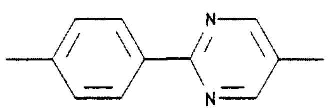 , 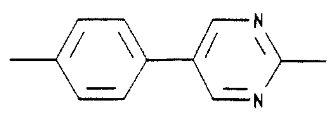 ,

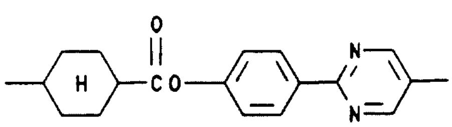 , 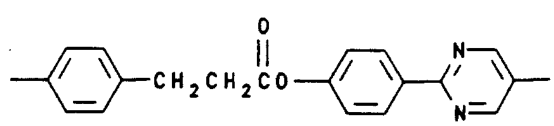

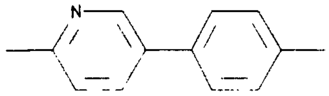 , 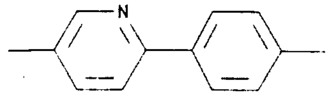 ,

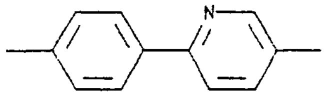 , 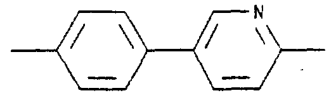 ,

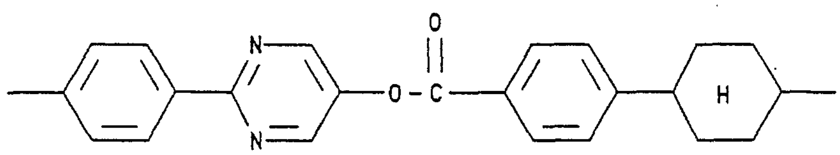 ,

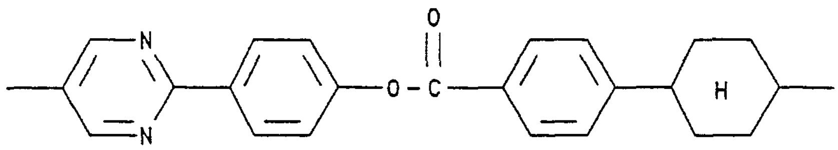 ,

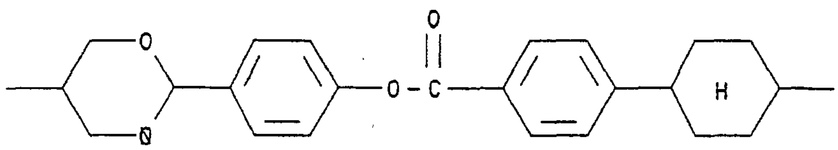 ,

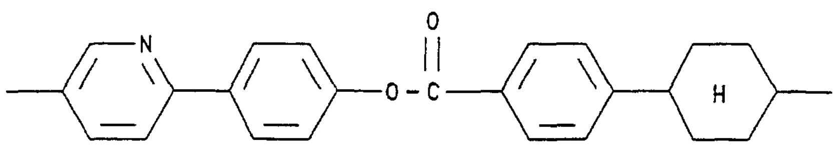 ,

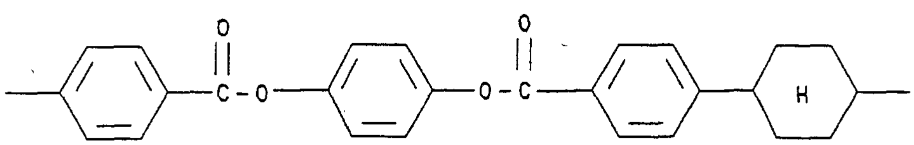

Die Herstellung der erfindungsgemäß verwendeten Verbindungen kann nach an sich literaturbekannten Methoden (siehe z. B. Houben-Weyl, Methoden der Organischen Chemie, Georg-Thieme-Verlag, Stuttgart; K. Dimitrowa, J. Hauschild, H. Zaschke und H. Schubert, Journal für praktische Chemie Bd. 322 (1980), Seite 933; H. Zaschke und H. Schubert, Journal für praktische Chemie Bd. 315 (1973), Seite 315) erfolgen.

Die erfindungsgemäß verwendeten Verbindungen der allgemeinen Formel I sind als Komponenten für ferroelektrische Flüssigkristallmischungen geeignet. Dabei können die LC-Mischungen 0,01 bis 60 Gew.-%, bevorzugt 0,1 bis 40 Gew.-%, besonders bevorzugt 0,1 bis 20 Gew.-% der erfindungsgemäß verwendeten Verbindungen enthalten. Die anderen Bestandteile werden vorzugsweise ausgewählt aus den bekannten Verbindungen mit nematischen, cholesterischen und/oder smektischen Phasen; dazu gehören beispielsweise Schiffsche Basen, Biphenyle, Terphenyle, Phenylcyclohexane, Cyclohexylbiphenyle, N, S oder O-haltige Heterocyclen, z. B. Pyrimidine, Zimtsäureester, Cholesterinester, oder verschieden überbrückte, terminalpolar mehrkernige Ester von p-Alkylbenzoesäuren.

Es zeigte sich nun überraschenderweise, daß durch Zugabe von Verbindungen der allgemeinen Formel I die Schaltgeschwindigkeit dieser Flüssigkristallmischungen erheblich gesteigert werden kann.

Diese Mischungen wiederum können Anwendung finden in elektrooptischen oder vollständig optischen Elementen, z.B. Anzeigeelementen, Schalteelementen, Lichtmodulatoren, Elementen zur Bildbearbeitung, Signalverarbeitung oder allgemein im Bereich der nichtlinearen Optik.

Die Erfindung wird durch die nachfolgenden Beispiele näher erläutert:
Für die ferroelektrischen Flüssigkristallmischungen wurden die Werte für die spontane Polarisation $P_s$[nC/cm$^2$] und die elektrische Schaltzeit τ[μs] bei einer Temperatur von 25°C gemessen.

Die $P_s$-Werte wurden nach der Methode von H. Diamant et al. (Rev. Sci. Instr., 28, 30, 1957) gemessen, wobei Meßzellen mit 10 μm Elektrodenabstand ohne Orientierungsschicht verwendet wurden.

Die Phasenumwandlungstemperaturen wurden beim Aufheizen mit Hilfe eines Polarisationsmikroskops anhand der Texturänderungen bestimmt. Die Bestimmung des Schmelzpunkts wurde hingegen mit einem DSC-Gerät durchgeführt. Die Angabe der Phasenumwandlungstemperaturen zwischen den Phasen

| | |
|---|---|
| Nematisch | N bzw. N*) |
| Smektisch-C | ($S_C$ bzw. $S_C$*) |
| Smektisch-A | ($S_A$) |
| Kristallin | (X) |

erfolgte in °C und die Werte stehen zwischen den Phasenbezeichnungen in der Phasenfolge.

Beispiel 1

2-(4-Decyloxyphenyl)-5-phenyl-(1,3,4)-thiadiazol

Die Synthese erfolgte analog der von K. Dimitrowa, J. Hauschild, H. Zaschke und H. Schubert im Journal für praktische Chemie Bd. 322 (1980), Seite 933 beschriebenen Methode.

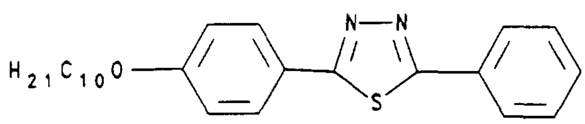

Die Verbindung zeigt folgende Phasenfolge:
X 99 $S_A$ 128 I

Beispiel 2

2-(4-Decylphenyl)-5-phenyl-(1,3,4)-thiadiazol

Die Synthese erfolgte analog Beispiel 1.

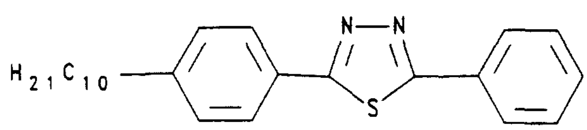

Die Verbindung zeigt folgende Phasenfolge:
X 70 $X_1$ 81 $S_A$ 88 I

Beispiel 3

2-(4-Nonylphenyl)-5-phenyl-(1,3,4)-thiadiazol

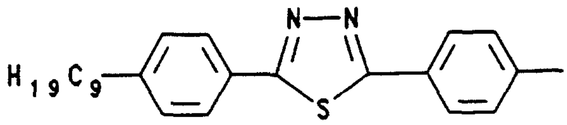

Die Synthese erfolgte analog Beispiel 1.

Die Verbindung zeigt folgende Phasenfolge:
X 81 $S_A$ 87 N 87,4 I

Beispiel 4

2-(4-Octyloxyphenyl)-5-phenyl-(1,3,4)-thiadiazol

Die Synthese erfolgte analog Beispiel 1.

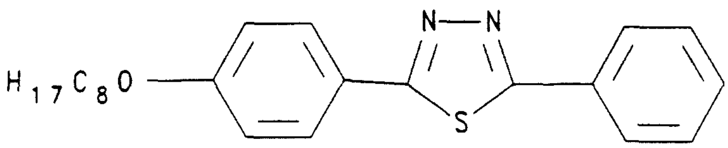

Die Verbindung zeigt folgende Phasenfolge:
X 103 $S_A$ 124 N 125 I

Beispiel 5

2-(4-Nonyloxyphenyl)-5-phenyl-(1,3,4)-thiadiazol

Die Synthese erfolgte analog Beispiel 1.

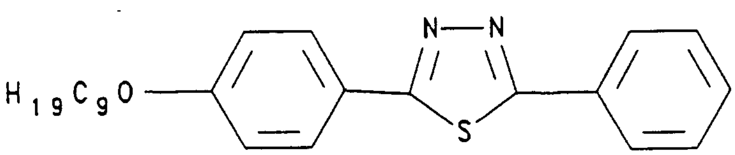

Die Verbindung zeigt folgende Phasenfolge:
X 97 $S_A$ 127 I

Beispiel 6

2-(4-Dodecylphenyl)-5-phenyl-(1,3,4)-thiadiazol

Die Synthese erfolgte analog Beispiel 1.

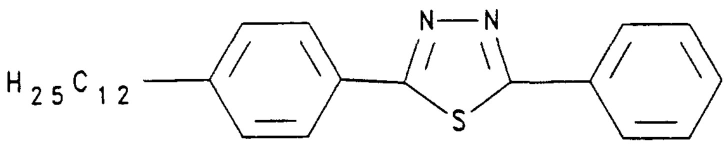

Die Verbindung zeigt folgende Phasenfolge:
X 67 $S_A$ 89 I

Beispiel 7

2-(4-Dodecyloxyphenyl)-5-phenyl-(1,3,4)-thiadiazol

Die Synthese erfolgte analog Beispiel 1.

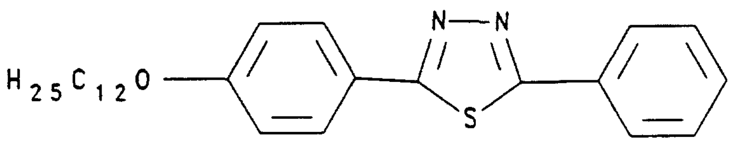

Die Verbindung zeigt folgende Phasenfolge:
X 84 $S_A$ 127 I

Beispiel 8

2-(4-Hexadecyloxyphenyl)-5-phenyl-(1,3,4)-thiadiazol

Die Synthese erfolgte analog Beispiel 1.

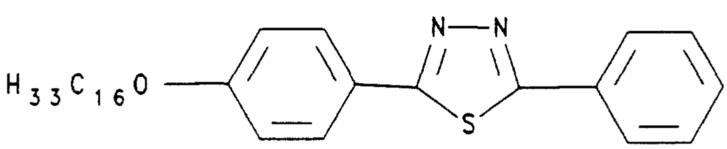

Die Verbindung zeigt folgende Phasenfolge:
X 75 $S_A$ 125 I

Beispiel 9

2-(4-Butyloxyphenyl)-5-phenyl-(1,3,4)-thiadiazol

Die Synthese erfolgte analog Beispiel 1.

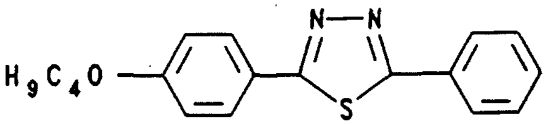

Die Verbindung zeigt die Phasenfolge:
X 97 $S_x$ 87 $S_C$ 89 $S_A$ 95 N 108 I

Beispiel 10

2-(4-Hexyloxyphenyl)-5-phenyl(1,3,4)-thiadiazol

Die Synthese erfolgte analog Beispiel 1.

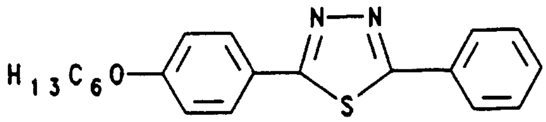

Die Verbindung zeigt die Phasenfolge:
$X_1$ 89 $X_2$ 94 $S_A$ 111 N 119 I

Beispiel 11

2-Phenyl-5-(4-tetradecyloxyphenyl)-(1,3,4)-thiadiazol

Die Synthese erfolgte analog Beispiel 1.

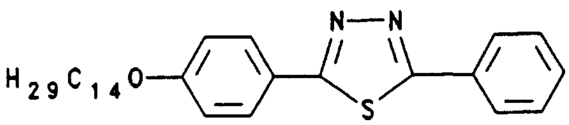

Die Verbindung zeigt die Phasenfolge:
X 90 $S_A$ 131 I

Beispiel 12

2-Cyclohexyl-5-(4-nonyloxyphenyl)-(1,3,4)-thiadiazol

Die Synthese erfolge analog Beispiel 1.

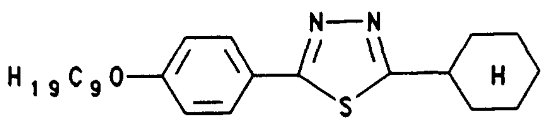

Die Verbindung zeigt die Phasenfolge:
$X_1$ 77 $X_2$ 86 $S_A$ 67 I

Beispiel 13

2-(4-Cyclohexylphenyl)-5-(4-nonyloxyphenyl)-(1,3,4)-thiadiazol

Die Synthese erfolgte analog Beispiel 1.

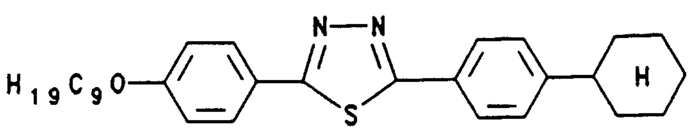

Die Verbindung zeigt die Phasenfolge:

S 126 $S_C$ 146 N 186 I

Beispiel 14

2-(4-Octyloxyphenyl)-5-phenylpyrimidin

Die Synthese erfolgte wie von H. Zaschke und H. Schubert im Journal für praktische Chemie Bd. 315 (1973), Seite 315 beschrieben.

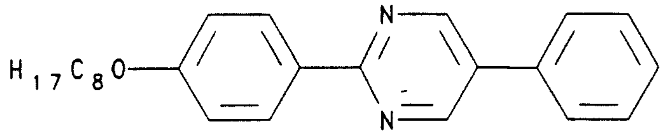

Die Verbindung zeigt folgende Phasenfolge:
X 100 N 113 I

Beispiel 15

2-(4-Dodecyloxyphenyl)-5-phenylpyrimidin

Die Synthese erfolgte analog Beispiel 14.

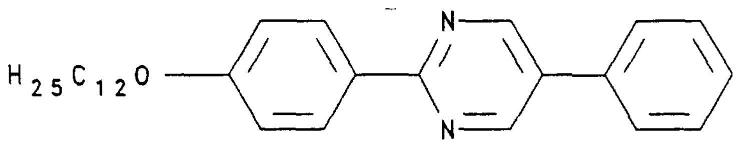

Die Verbindung zeigt folgende Phasenfolge:
X 103 N 109 I

Beispiel 16

5-(4-Dodecyloxyphenyl)-2-phenylpyrimidin.

Die Synthese erfolgt analog Beispiel 14.

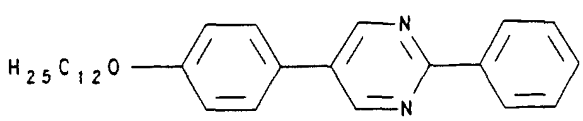

Die Verbindung zeigt folgende Phasenfolge:
X 93 $S_A$ 156 I

Beispiel 17

5-(4-Hexyloxyphenyl)-2-phenylpyrimidin

Die Synthese erfolgte analog Beispiel 14.

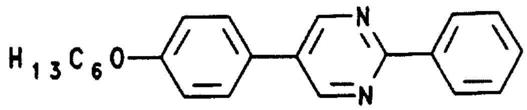

Die Verbindung zeigt die Phasenfolge:
X 92 $S_A$ 163 I

Beispiel 18

5-(4-Hexylphenyl)-2-phenylpyrimidin

Die Synthese erfolgte analog Beispiel 14.

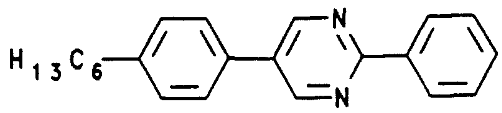

Die Verbindung zeigt die Phasenfolge:
X 97 $S_A$ 133 I

Beispiel 19

4-Cyclohexylphenylcarbonsäure-2-(4-octyloxyphenyl)pyrimidin-5-yl-ester

2,00 g (6,66 mmol) 2-(4-Octyloxyphenyl)-5-hydroxypyrimidin, 1,36 g (6,66 mmol) 4-Cyclohexylphenylcarbonsäure, 1,37 g (6,66 mmol) Dicyclohexylcarbodiimid und eine Spatelspitze N,N-Dimethylaminopyridin wurden 17 h bei Raumtemperatur gerührt. Anschließend wurde filtriert, das Filtrat eingeengt und der Rückstand über Kieselgel mit Hexan : Essigester = 8 : 2 chromatographiert. Nach Umkristallisation aus Hexan wurden 2,13 g 4-Cyclohexylphenylcarbonsäure-2-(4-octyloxyphenyl)pyrimidin-5-yl-ester erhalten.

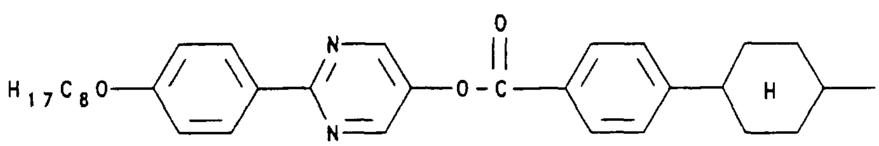

Die Verbindung zeigt folgende Phasenfolge:
X 108 206 I

Beispiel 20

4-Cyclohexylphenylcarbonsäure-4-(5-octyl-1,3-dioxan-2-yl)phenyl-ester

Die Synthese erfolgte analog Beispiel 19.

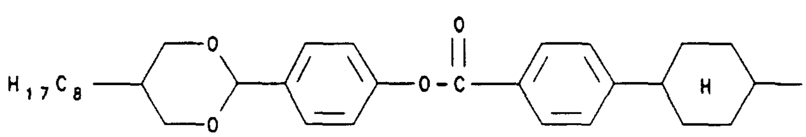

Die Verbindung zeigt folgende Phasenfolge:
X 106 N 169 I

Beispiel 21

4-Cyclohexylphenylcarbonsäure-4-(5-octylpyridin-2-yl)phenyl-ester

Die Synthese erfolgte analog Beispiel 19.

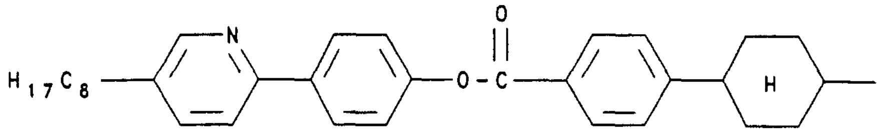

Die Verbindung zeigt folgende Phasenfolge:
X 133 N 182 I

Beispiel 22

4-Cyclohexylphenylcarbonsäure-4-(5-octyloxypyrimidin-2-yl)phenyl-ester

Die Synthese erfolgte analog Beispiel 19.

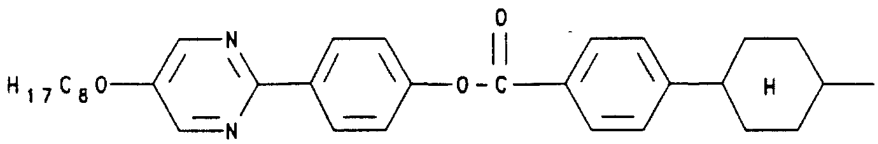

Die Verbindung zeigt folgende Phasenfolge:
X 144 N 200 I

Beispiel 23

4-Cyclohexylphenylcarbonsäure-4-(4-octyloxyphenylcarbonyloxy)phenyl-ester

Die Synthese erfolgte analog Beispiel 19.

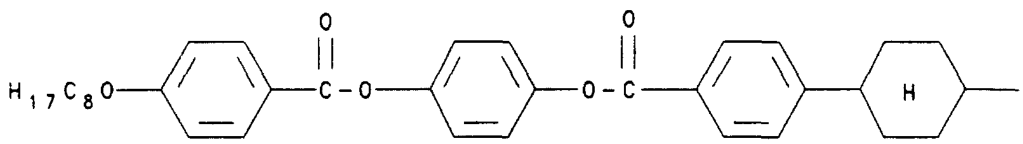

Die Verbindung zeigt folgende Phasenfolge:
X 146 N 212 I

Beispiel 24

Trans-4-pentylcyclohexancarbonsäure-4-(pyrimidin-2-yl)phenyl-ester

Die Synthese erfolgte analog Beispiel 19.

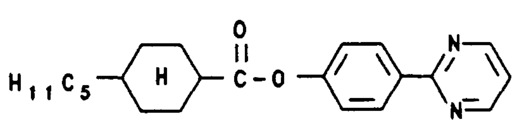

Die Verbindung zeigt die Phasenfolge:
X 117 I

Beispiel 25

3-(4-Ethylphenyl)propansäure-4-(pyrimidin-2-yl)phenyl-ester

Die Synthese erfolgte analog Beispiel 19.

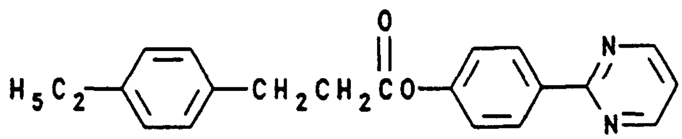

Die Verbindung zeigt die Phasenfolge:
X 113 I

Beispiel 26

2-(4-Octyloxyphenyl)pyrimidin

0,86 g (5,00 mmol) 2-(4-Hydroxyphenyl)pyrimidin werden in 40 ml Dimethylformamid portionsweise mit 0,30 g (7,50 mmol) 60 %igem Natriumhydrid versetzt und 15 min bei Raumtemperatur gerührt. Anschließend werden 1,45 g (7,50 mmol) 1-Bromoctan zugegeben, über Nacht gerührt, auf Eiswasser gegossen, filtriert, und der Rückstand chromatographisch (Kieselgel/Dichlormethan) und durch Umkristallisation aus Acetonitril gereinigt. Es werden 0,93 g 2-(4-Octyloxyphenyl)pyrimidin erhalten.

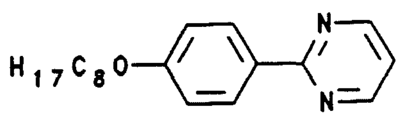

Die Verbindung zeigt die Phasenfolge:
X 51 I

Beispiel 27

5-(2-Octyloxypyrimid-5-yl)-2-phenylpyridin

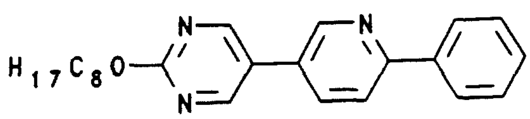

Die Synthese erfolgte analog der Vorschriften in Tetrahedron Letters 28 (1987) 5093 oder Mol. Cryst. Liq. Cryst. 204 (1991) 91 oder Mol. Cryst. Liq. Cryst. 206 (1991) 187 oder J. Chem. Soc. Perkin. Trans. II 1989, 2041 aus 2-Octyloxy-5-brompyrimidin und 2-Phenylpyridin-5-boronsäure.

Die Verbindung zeigt folgende Phasenfolge:
X 133 $S_A$ 153 I

Beispiel 28

5-[2-(9-Decenyloxy)pyrid-5-yl]-2-phenylpyrimidin

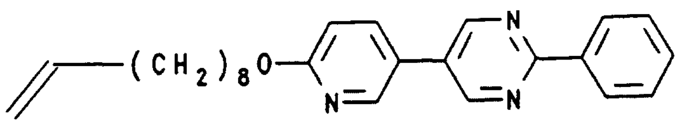

Die Synthese erfolgte analog Beispiel 27 aus 2-Phenyl-5-brompyrimidin und 2-Decenyloxypyridin-5-boronsäure.

Die Verbindung zeigt folgende Phasenfolge:

X 87 $S_A$ 125 I

Beispiel 29

5-(2-Decyloxypyrimid-5-yl)-2-phenylpyridin

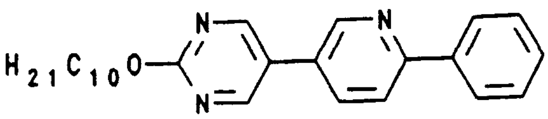

Die Synthese erfolgte analog Beispiel 27 aus 2-Decenyloxy-5-brompyrimidin und 2-Phenylpyridin-5-boronsäure.

Die Verbindung zeigt folgende Phasenfolge:

X 125 $S_A$ 154 I

Beispiel 30

2-[2-(9-Decenyloxy)pyrid-5-yl]-5-phenylpyrimidin

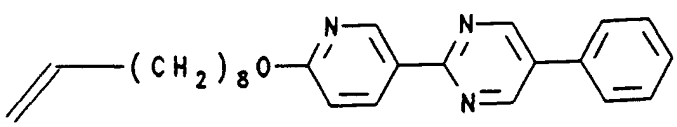

Die Synthese erfolgte analog Beispiel 27 aus 2-Brom-5-phenylpyrimidin und 2-Decenylpyridin-5-boronsäure.

Die Verbindung zeigt folgende Phasenfolge:

X 96 $S_A$ 91 I

Beispiel 31

5-(5-Dodecylpyrimid-2-yl)-2-phenylpyridin

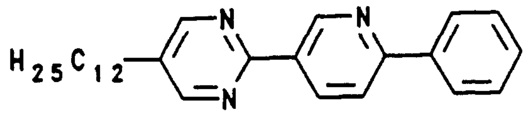

Die Synthese erfolgte analog Beispiel 27 aus 2-Brom-5-dodecylpyrimidin und 2-Phenylpyridin-5-boronsäure.
Die Verbindung zeigt folgende Phasenfolge:

X 76 $S_A$ 130 I

Anwendungsbeispiel 1

Die Mischung besteht aus den Komponenten

| | | |
|---|---|---|
| 1 | 5-Octyloxy-2-(4-hexyloxy-phenyl)-pyrimidin | 7,8 Mol-% |
| 2 | 5-Octyloxy-2-(4-butyloxy-phenyl)-pyrimidin | 8,3 Mol-% |
| 3 | 5-Octyloxy-2-(4-decyloxy-phenyl)-pyrimidin | 6,5 Mol-% |
| 4 | 5-Octyloxy-2-(4-octyloxy-phenyl)-pyrimidin | 3,6 Mol-% |
| 5 | 5-Octyl-2-(4-hexyloxy-phenyl)-pyrimidin | 12,5 Mol-% |
| 6 | 5-Octyl-2-(4-octyloxy-phenyl)-pyrimidin | 11,2 Mol-% |
| 7 | 5-Octyl-2-(4-decyloxy-phenyl)-pyrimidin | 8,3 Mol-% |
| 8 | trans-4-Pentyl-cyclohexancarbonsäure-(4-(5-dodecylpyrimidin-2-yl)-phenylester | 12,3 Mol-% |
| 9 | (2S,3S)-2-[4-(5-Octyl-pyrimidin-2-yl)-phenyloxy]-methyl-3-butyl-oxiran | 5,7 Mol-% |
| 10 | (2R,3R)-3-Propyl-oxiran-2-carbonsäure-(4-(2-octyloxy-pyrimidin-5-yl)-phenyl)-ester | 6,5 Mol-% |
| 11 | (S)-4-(2-Octyloxypyrimidin-5-yl)-phenyl-(spiro-(1,3-dioxolan-2,1-cyclohexan)-4-yl)-methylether | 3,4 Mol-% |
| 12 | 2-(4-Nonyl-phenyl)-5-phenyl-1,3,4-thiadiazol | 3,0 Mol-% |
| 13 | 2-(4-Decyl-phenyl)-5-phenyl-1,3,4-thiadiazol | 3,0 Mol-% |
| 14 | 2-(4-Octyloxy-phenyl)-5-phenyl-pyrimidin | 3,9 Mol-% |
| 15 | 2-(4-Decyloxy-phenyl)-5-phenyl-1,3,4-thiadiazol | 2,5 Mol-% |
| 16 | 4,7,13,16,21,24-Hexaoxa-1,10-diazabicyclo(8.8.8)hexacosan (®Kryptofix 222) | 1,5 Mol-% |

Die Mischung zeigt folgende flüssigkristalline Phasenbereiche:

X -9 $S_c^*$ 56 $S_A$ 70 N* 82 I

Die gebrauchsfertige ferroelektrische Mischung wurde in eine 2µm dicke, mit Polyimid beschichtete Zelle gefüllt und 10 Minuten lang einer Rechteckfeldbehandlung von 15V/µm bei einer Frequenz von 10 Hz ausgesetzt. Anschließend schaltete die Probe bei einer angelegten Feldstärke von 10V/µm mit einer Pulsbreite von 91 µs.
Eine Flüssigkristallmischung, die sich nur dadurch von der oben genannten unterscheidet, daß die Komponenten 12-15 (erfindungsgemäße Verbindungen) nicht zugesetzt wurden, zeigt die Phasenbereiche:

X -4 $S_c^*$ 58 $S_A$ 67 N* 82 I

Die minimale Pulsdauer, bei der die Probe bei einer Feldstärke von 10 Vµm schalten, beträgt 115 µs.
Werden 1,5 Mol-% der Komponente 16 gegen 0,5 Mol-% 1-(Tert.-butylcarbonyl)-1-aza-4,7,10,13-tetraoxacyclopentadecan(13-1,4,7,10) ausgetauscht, so schaltet die Probe bei einer Feldstärke von 18 V/µm mit einer Pulsbreite von 50 µs.

Anwendungsbeispiel 2

Die Mischung besteht aus den Komponenten

| | | |
|---|---|---|
| 1 | 5-Octyloxy-2-(4-hexyloxy-phenyl)-pyrimidin | 8,6 Mol-% |
| 2 | 5-Octyloxy-2-(4-butyloxy-phenyl)-pyrimidin | 9,1 Mol-% |
| 3 | 5-Octyloxy-2-(4-decyloxy-phenyl)-pyrimidin | 7,1 Mol-% |
| 4 | 5-Octyloxy-2-(4-octyloxy-phenyl)-pyrimidin | 4,0 Mol-% |
| 5 | 5-Octyl-2-(4-hexyloxy-phenyl)-pyrimidin | 13,7 Mol-% |
| 6 | 5-Octyl-2-(4-octyloxy-phenyl)-pyrimidin | 12,3 Mol-% |
| 7 | 5-Octyl-2-(4-decyloxy-phenyl)-pyrimidin | 9,1 Mol-% |
| 8 | trans-4-Pentyl-cyclohexancarbonsäure(4-(5-dodecylpyrimidin-2-yl)-phenylester | 13,5 Mol-% |
| 9 | (2S,3S)-2-[4-(5-Octyl-pyrimidin-2-yl)phenyloxy]-methyl-3-butyl-oxiran | 6,3 Mol-% |
| 10 | (2R,3R)-3-Propyl-oxiran-2-carbonsäure(4-(2-octyloxy-pyrimidin-5-yl)-phenyl)-ester | 7,1 Mol-% |
| 11 | (S)-4-(2-Octyloxypyrimdin-5-yl)-phenyl-(spiro-(1,3-dioxolan-2,1-cyclohexan)-4-yl)-methylether | 3,7 Mol-% |
| 12 | 5-(5-Dodecylpyrimid-1-yl)-2-phenylpyridin | 5,0 Mol-% |
| 13 | 1-(Tert.-butylcarbonyl)-1-aza-4,7,10,13-tetraoxacyclopentadecan(13-1,4,7,10) | 0,5 Mol-% |

Die Mischung zeigt folgende flüssigkristalline Phasenbereiche:

$S_c^*$ 57 $S_A$ 71 N* 81 I

Die gebrauchsfertige ferroelektrische Mischung wurde in eine 2 µm dicke, mit Polyimid beschichtete Zelle gefüllt und 10 Minuten lang einer Rechteckfeldbehandlung von 15 V/µm bei einer Frequenz von 10 Hz ausgesetzt. Anschließend schaltete die Probe bei einer angelegten Feldstärke von 15,2 V/µm mit einer Pulsbreite von 50 µs.

Anwendungsbeispiel 3

Die Mischung besteht aus den Komponenten

| | | |
|---|---|---|
| 1 | 5-Octyloxy-2-(4-hexyloxy-phenyl)-pyrimidin | 8,6 Mol-% |
| 2 | 5-Octyloxy-2-(4-butyloxy-phenyl)-pyrimidin | 9,1 Mol-% |
| 3 | 5-Octyloxy-2-(4-decyloxy-phenyl)-pyrimidin | 7,1 Mol-% |
| 4 | 5-Octyloxy-2-(4-octyloxy-phenyl)-pyrimidin | 4,0 Mol-% |
| 5 | 5-Octyl-2-(4-hexyloxy-phenyl)-pyrimidin | 13,7 Mol-% |
| 6 | 5-Octyl-2-(4-octyloxy-phenyl)-pyrimidin | 12,3 Mol-% |
| 7 | 5-Octyl-2-(4-decyloxy-phenyl)-pyrimidin | 9,1 Mol-% |
| 8 | trans-4-Pentyl-cyclohexancarbonsäure-(4-(5-dodecylpyrimidin-2-yl)-phenylester | 13,5 Mol-% |
| 9 | (2S,3S)-2-[4-(5-Octyl-pyrimidin-2-yl)-phenyloxy]-methyl-3-butyl-oxiran | 6,3 Mol-% |
| 10 | (2R,3R)-3-Propyl-oxiran-2-carbonsäure-(4-(2-octyloxy-pyrimidin-5-yl)phenyl)-ester | 7,1 Mol-% |
| 11 | (S)-4-(2-Octyloxypyrimdin-5-yl)-phenyl-(spiro-(1,3-dioxolan-2,1-cyclohexan)-4-yl)-methylether | 3,7 Mol-% |
| 12 | 2-(2-[9-decenyloxy]pyridin-5-yl)-2-phenylpyrimidin | 5,0 Mol-% |
| 13 | 1-(Tert.-butylcarbonyl)-1-aza-4,7,10,13-tetraoxacylopentadecan(13-1,4,7,10) | 0,5 Mol-% |

Die Mischung zeigt folgende flüssigkristalline Phasenbereiche:

$S_c^*$ 62 $S_A$ 76 N* 83 I

Die gebrauchsfertige ferroelektrische Mischung wurde in eine 2 µm dicke, mit Polyimid beschichtete Zelle gefüllt und 10 Minuten lang einer Rechteckfeldbehandlung von 15 V/µm bei einer Frequenz von 10 Hz ausgesetzt. Anschließend schaltete die Probe bei einer angelegten Feldstärke von 12,7 V/µm mit einer Pulsbreite von 50 µs.

Anwendungsbeispiel 4

Die Mischung besteht aus den Komponenten

| | | |
|---|---|---|
| 1 | 5-Octyloxy-2-(4-hexyloxy-phenyl)-pyrimidin | 8,6 Mol-% |
| 2 | 5-Octylory-2-(4-butyloxy-phenyl)-pyrimidin | 9,1 Mol-% |
| 3 | 5-Octyloxy-2-(4-decyloxy-phenyl)-pyrimidin | 7,1 Mol-% |

(fortgesetzt)

| | | |
|---|---|---|
| 4 | 5-Octyloxy-2-(4-octyloxy-phenyl)-pyrimidin | 4,0 Mol-% |
| 5 | 5-Octyl-2-(4-hexyloxy-phenyl)-pyrimidin | 13,7 Mol-% |
| 6 | 5-Octyl-2-(4-octyloxy-phenyl)-pyrimidin | 12,3 Mol-% |
| 7 | 5-Octyl-2-(4-decyloxy-phenyl)-pyrimidin | 9,1 Mol-% |
| 8 | trans-4-Pentyl-cyclohexancarbonsäure-(4-(5-dodecylpyrimidin-2-yl)-phenylester | 13,5 Mol-% |
| 9 | (2S,3S)-2-[4-(5-Octyl-pyrimidin-2-yl)-phenyloxy]-methyl-3-butyl-oxiran | 6,3 Mol-% |
| 10 | (2R,3R)-3-Propyl-oxiran-2-carbonsäure-(4-(2-octyloxy-pyrimidin-5-yl)-phenyl)-ester | 7,1 Mol-% |
| 11 | (S)-4-(2-Octyloxypyrimdin-5-yl)-phenyl-(spiro-(1,3-dioxolan-2,1-cyclohexan)-4-yl)-methylether | 3,7 Mol-% |
| 12 | 3-(4-Ethylcyclohexyl)propansäure-4-(pyrimidin-2-yl)-phenylester | 5,0 Mol-% |
| 13 | 1-(Tert.-butylcarbonyl)-1-aza-4,7,10,13-tetraoxacylopentadecan(13-1,4,7,10) | 0,5 Mol-% |

Die Mischung zeigt folgende flüssigkristalline Phasenbereiche:

$S_c^*$ 57 $S_A$ 63 N* 79 I

Die gebrauchsfertige ferroelektrische Mischung wurde in eine 2 µm dicke, mit Polyimid beschichtete Zelle gefüllt und 10 Minuten lang einer Rechteckfeldbehandlung von 15 V/µm bei einer Frequenz von 10 Hz ausgesetzt. Anschließend schaltete die Probe bei einer angelegten Feldstärke von 17,4 V/µm mit einer Pulsbreite von 50 µs.

Anwendungsbeispiel 5

Die Mischung besteht aus den Komponenten

| | | |
|---|---|---|
| 1 | 5-Octyloxy-2-(4-hexyloxy-phenyl)-pyrimidin | 8,7 Mol-% |
| 2 | 5-Octyloxy-2-(4-butyloxy-phenyl)-pyrimidin | 9,2 Mol-% |
| 3 | 5-Octyloxy-2-(4-decyloxy-phenyl)-pyrimidin | 7,2 Mol-% |
| 4 | 5-Octyloxy-2-(4-octyloxy-phenyl)-pyrimidin | 4,0 Mol-% |
| 5 | 5-Octyl-2-(4-hexyloxy-phenyl)-pyrimidin | 13,9 Mol-% |
| 6 | 5-Octyl-2-(4-octyloxy-phenyl)-pyrimidin | 12,4 Mol-% |
| 7 | 5-Octyl-2-(4-decyloxy-phenyl)-pyrimidin | 9,2 Mol-% |
| 8 | trans-4-Pentyl-cyclohexancarbonsäure-(4-(5-dodecylpyrimidin-2-yl)-phenylester | 13,6 Mol-% |
| 9 | (2S,3S)-2-[4-(5-Octyl-pyrimidin-2-yl)-phenyloxy]-methyl-3-butyl-oxiran | 6,3 Mol-% |
| 10 | (2R,3R)-3-Propyl-oxiran-2-carbonsäure-(4-(2-octyloxy-pyrimidin-5-yl)-phenyl)-ester | 7,2 Mol-% |
| 11 | (S)-4-(2-Octyloxypyrimdin-5-yl)-phenyl-(spiro-(1,3-dioxolan-2,1-cyclohexan)-4-yl)-methylether | 3,8 Mol-% |
| 12 | 2-(pyridin-4-yl)-5-(4-decyloxyphenyl)-pyrimidin | 4,0 Mol-% |
| 13 | 1-(Tert.-butylcarbonyl)-1-aza-4,7,10,13-tetraoxacylopentadecan(13-1,4,7,10) | 0,5 Mol-% |

Die Mischung zeigt folgende flüssigkristalline Phasenbereiche:

$S_c^*$ 55 $S_A$ 79 N* 84 I

Die gebrauchsfertige ferroelektrische Mischung wurde in eine 2 µm dicke, mit Polyimid beschichtete Zelle gefüllt und 10 Minuten lang einer Rechteckfeldbehandlung von 15 V/µm bei einer Frequenz von 10 Hz ausgesetzt. Anschließend schaltete die Probe bei einer angelegten Feldstärke von 10,4 V/µm mit einer Pulsbreite von 50 µs.

Anwendungsbeispiel 6

Die Mischung besteht aus den Komponenten

| | | |
|---|---|---|
| 1 | 5-Octyloxy-2-(4-hexyloxy-phenyl)-pyrimidin | 8,1 Mol-% |
| 2 | 5-Octyloxy-2-(4-butyloxy-phenyl)-pyrimidin | 8,6 Mol-% |
| 3 | 5-Octyloxy-2-(4-decyloxy-phenyl)-pyrimidin | 6,8 Mol-% |
| 4 | 5-Octyloxy-2-(4-octyloxy-phenyl)-pyrimidin | 3,7 Mol-% |
| 5 | 5-Octyl-2-(4-hexyloxy-phenyl)-pyrimidin | 13,0 Mol-% |

(fortgesetzt)

| | | |
|---|---|---|
| 6 | 5-Octyl-2-(4-octyloxy-phenyl)-pyrimidin | 11,6 Mol-% |
| 7 | 5-Octyl-2-(4-decyloxy-phenyl)-pyrimidin | 8,6 Mol-% |
| 8 | trans-4-Pentyl-cyclohexancarbonsäure-(4-(5-dodecylpyrimidin-2-yl)-phenylester | 12,8 Mol-% |
| 9 | (2S,3S)-2-[4-(5-Octyl-pyrimidin-2-yl)-phenyloxy]-methyl-3-butyl-oxiran | 5,9 Mol-% |
| 10 | (2R,3R)-3-Propyl-oxiran-2-carbonsäure-(4-(2-octyloxy-pyrimidin-5-yl)-phenyl)-ester | 6,7 Mol-% |
| 11 | (S)-4-(2-Octyloxypyrimdin-5-yl)-phenyl-(spiro-(1,3-dioxolan-2,1-cyclohexan)-4-yl)-methylether | 3,5 Mol-% |
| 12 | 2-(4-Nonyl-phenyl)-5-phenyl-1,3,4-oxadiazol | 10,0 Mol-% |
| 13 | 1-(Tert.-butylcarbonyl)-1-aza-4,7,10,13-tetraoxacylopentadecan(13-1,4,7,10) | 0,5 Mol-% |

Die Mischung zeigt folgende flüssigkristalline Phasenbereiche:

$S_c^*$ 58 $S_A$ 65 N* 77 I

Die gebrauchsfertige ferroelektrische Mischung wurde in eine 2 µm dicke, mit Polyimid beschichtete Zelle gefüllt und 10 Minuten lang einer Rechteckfeldbehandlung von 15 V/µm bei einer Frequenz von 10 Hz ausgesetzt. Anschließend schaltete die Probe bei einer angelegten Feldstärke von 14,5 V/µm mit einer Pulsbreite von 50 µs.
Der Vergleich der Schaltfelder für die Mischungen der Anwendungsbeispiele 2-6 mit dem Schaltfeld der im Anwendungsbeispiel 1 genannten Vergleichsmischung belegt, daß durch den Einsatz der erfindungsgemäßen Verbindungen eine erhebliche Minderung der zum Schalten benötigten Feldstärke erreicht werden kann.

Anwendungsbeispiel 7

Eine ferroelektrische Mischung besteht aus den Komponenten

| | |
|---|---|
| 2-(4-Decyloxy-phenyl)-5-phenyl-1,3,4-thiadiazol | 10,0 Mol-% |
| 5-Octyl-2-(4-hexyloxy-phenyl)-pyrimidin | 11,2 Mol-% |
| 5-Octyl-2-(4-decyloxy-phenyl)-pyrimidin | 7,5 Mol-% |
| 5-Decyl-2-(4-hexyloxy-phenyl)-pyrimidin | 7,2 Mol-% |
| 5-Octyl-2-(4-(7-cyclopropylheptyloxy)phenyl)-pyridin | 6,0 Mol-% |
| 5-Octyl-2-(4-(6-cyclopropyl)-hexyl-carbonyloxy-phenyl)-pyrimidin | 7,5 Mol-% |
| 5-(8-Cyclopropyloctyloxy)-2-(4-trans-pentyl-cyclohexyl-4-phenyl)-pyrimidin | 8,7 Mol-% |
| trans-4-Pentyl-cyclohexancarbonsäure-4-(8-cyclopropyloctyl)-pyrimidin-2-yl-phenylester | 5,3 Mol-% |
| 5-(5-Cyclopropylpentyloxy)-2-(4-hexyloxy-phenyl)-pyrimidin | 8,0 Mol-% |
| (2S,3S)-2-(4-(5-(9-Decenyloxy-pyrimidin-2-yl)-phenyloxy)-methyl-3-butyl-oxiran | 11,5 Mol-% |
| (2S,3S)-2-(4-(5-(7-Octenyloxy-pyrimidin-2-yl)-phenyloxy)-methyl-3-butyl-oxiran | 3,4 Mol-% |
| (2S,3S)-2-(4-(5-(5-Hexenyloxy)-pyrimidin-2-yl)-phenyloxy)-methyl-3-butyl-oxiran | 3,4 Mol-% |
| (S)-4-(5-Octyloxypyrimidin-2-yl)-phenyl-2,2-dimethyl-1,3-dioxolan)-4-yl)-methyl-ether | 1,7 Mol-% |
| (2R,3R)-3-Propyl-oxiran-2-carbonsäure-(4-(2-undecyloxy-pyrimidin-5-yl)-phenyl)-ester | 6,6 Mol-% |
| Kronenether (18 Krone 6) | 2,0 Mol-% |

Die Mischung zeigt folgende flüssigkristalline Phasenbereiche:

$S_c^*$ 60 $S_A$ 74 N* 83 I

und weist bei 25°C eine spontane Polarisation von 41 nC/cm$^2$ auf. Die Mischung schaltet bei 25°C in einer 2 µm dicken Zelle in einem elektrischen Feld von 10 V/µm mit einer Schaltzeit von 58 µsec.

Anwendungsbeispiel 8

Eine ferroelektrische Mischung besteht aus den Komponenten

| | |
|---|---|
| 2-(4-Decyl-phenyl)-5-phenyl-1,3,4-thiadiazol | 10,0 Mol-% |
| 5-Octyl-2-(4-hexyloxy-phenyl)-pyrimidin | 11,2 Mol-% |
| 5-Octyl-2-(4-decyloxy-phenyl)-pyrimidin | 7,5 Mol-% |
| 5-Decyl-2-(4-hexyloxy-phenyl)-pyrimidin | 7,2 Mol-% |

(fortgesetzt)

| | |
|---|---|
| 5-Octyl-2-(4-(7-cyclopropylheptyloxy)-phenyl)-pyridin | 6,0 Mol-% |
| 5-Octyl-2-(4-(6-cyclopropyl)-hexylcarbonyloxy-phenyl)-pyrimidin | 7,5 Mol-% |
| 5-(8-Cyclopropyloctyloxy)-2-(4-trans-pentyl-cyclohexyl-4-phenyl)-pyrimidin | 8,7 Mol-% |
| trans-4-Pentyl-cyclohexancarbonsäure-4-(8-Cyclopropyloctyl)-pyrimidin-2-yl-phenylester | 5,3 Mol-% |
| 5-(5-cyclopropylpentyloxy-2-(4-hexyloxy-phenyl)-pyrimidin | 8,0 Mol-% |
| ((2S,3S)-2-(4-(5-(9-Decenyloxy-pyrimidin-2-yl)-phenyloxy)-methyl-3-butyl-oxiran | 11,5 Mol-% |
| (2S,3S)-2-(4-(5-(7-Octenyloxy-pyrimidin-2-yl)-phenyloxy)-methyl-3-butyl-oxiran | 3,4 Mol-% |
| (2S,3S)-2-(4-(5-(5-Hexenyloxy-pyrimidin-2-yl)-phenyloxy)-methyl-3-butyl-oxiran | 3,4 Mol-% |
| (S)-4-(5-Octyloxypyrimidin-2-yl)-phenyl-2,2-dimethyl-1,3-dioxolan)-4-yl)-methyl-ether | 1,7 Mol-% |
| (2R,3R)-3-Propyl-oxiran-2-carbonsäure-(4-(2-undecyloxy-pyrimidin-5-yl)-phenyl)-ester | 6,6 Mol-% |
| Kronenether (18 Krone 6) | 2,0 Mol-% |

Die Mischung zeigt folgende flüssigkristalline Phasenbereiche:

$S_c^*$ 56 $S_A$ 68 N* 80 I

und weist bei 25°C eine spontane Polarisation von 38 $nC/cm^2$ auf. Die Mischung schaltet bei 25°C in einer 2 µm dicken Zelle in einem elektrischen Feld von 10 V/µm mit einer Schaltzeit von 63 µsec.

Eine Flüssigkristallmischung, die sich nur dadurch von den beiden letztgenannten Mischungen unterscheidet, daß die erste (erfindungsgemäße) Komponente nicht zugesetzt wurde, zeigt die Phasenbereiche:

$S_c^*$ 58 $S_A$ 67 N* 78 I

Die Polarisation beträgt 46 $nC/cm^2$, die Schaltzeit beträgt 73 µs bei einer Feldstärke von 10 $V\mu m^{-1}$.

Diese Beispiele belegen, daß mit den erfindungsgemäßen Komponenten ferroelektrische Mischungen hergestellt werden können, die sich durch kurze Schaltzeiten auszeichnen.

## Patentansprüche

**1.** Verwendung von Verbindungen der allgemeinen Formel I

$$R^1(-A^1)_a(-M^1)_b(-A^2)_c(-M^2)_d(-A^3)_e(-M^3)_1(-A^4)_g\text{-}H \qquad (I)$$

in der die Symbole und Indices folgende Bedeutung haben:

$R^1$ ist ein geradkettiger oder verzweigter Alkylrest mit 1 bis 22 C-Atomen (mit oder ohne asymmetrisches C-Atom), wobei auch eine oder zwei nicht benachbarte $-CH_2$-Gruppen durch -O-, -S-, -CO-, - CO-O-, -O-CO-, -CO-S-, -S-CO-, -O-CO-O-, -CH=CH-, -C≡C-, oder $-Si(CH_3)_2$- ersetzt sein können und wobei auch ein oder mehrere H-Atome des Alkylrestes durch F, Cl, Br oder CN substituiert sein können, oder ist eine der nachfolgenden chiralen Gruppen:

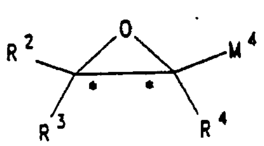
,
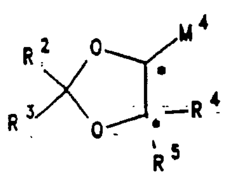
,

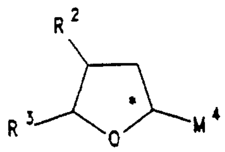 ,

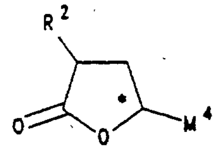 ,

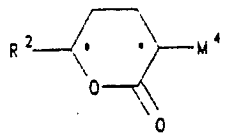 ,

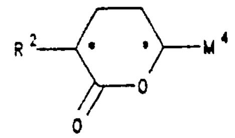 ,

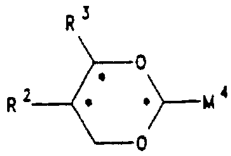 ,

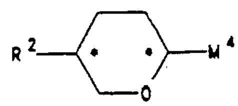 ,

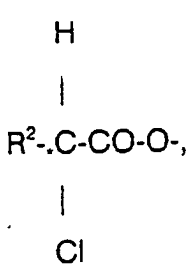

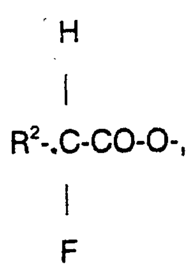

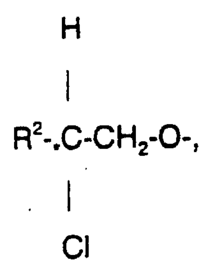

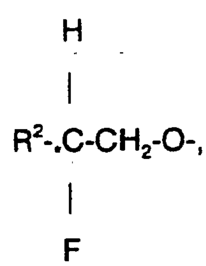

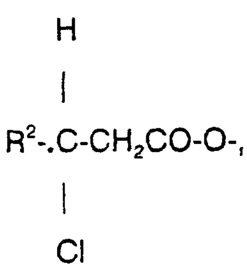

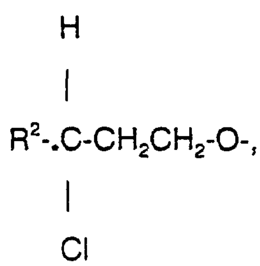

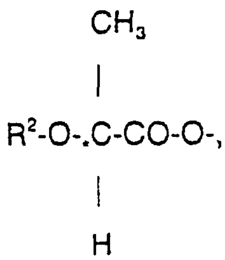

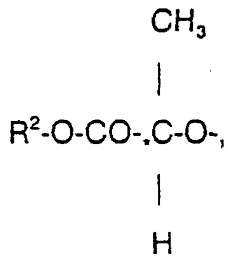

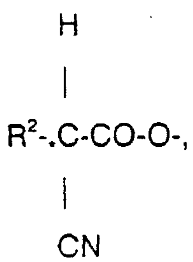

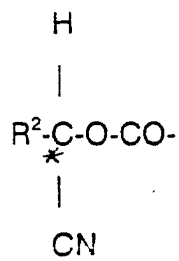

$R^2$, $R^3$, $R^4$, $R^5$ sind unabhängig voneinander H oder ein geradkettiger oder verzweigter Alkylrest mit 1 bis 22 C-Atomen, wobei auch eine oder zwei nicht benachbarte-$CH_2$- Gruppen durch -O-, -S-, -CO-, -CO-O-, -O-CO-, -CO-S-, -S-CO-, -O-CO-O-, -CH=CH-, -C≡C-, oder -$Si(CH_3)_2$- ersetzt sein können, oder $R^2$ und $R^3$ können zusammen auch -$(CH_2)_4$- oder -$(CH_2)_5$- sein, wenn sie als Substituenten an ein Dioxolan - System gebunden sind,

$A^1$, $A^2$, $A^3$, $A^4$ sind gleich oder verschieden 1,4-Phenylen, wobei ein oder zwei H-Atome durch F, Cl und/oder CN ersetzt sein können, Pyrazin-2,5-diyl, Pyridazin-3,6-diyl, Pyridin-2,5-diyl, Pyrimidin-2,5-diyl, bei dem ein oder zwei H-Atome durch F ersetzt sein können, trans-1,4-Cyclohexylen, (1,3,4)-Thiadiazol-2,5-diyl, 1,3-Dioxan-2,5-diyl, 1,3-Dithian-2,5-diyl, 1,3-Thiazol-2,4-diyl, 1,3 - Thiazol - 2,5 - diyl, Thiophen-2,4-diyl, Thiophen-2,5-diyl, Piperazin-1,4-diyl, Piperazin-2,5-diyl, Naphthalin-2,6-diyl, Bicyclo[2.2.2]octan-1,4-diyl, 1,3-Dioxaborinan-2,5-diyl oder trans- Dekalin-2,6- diyl,

$M^1$, $M^2$, $M^3$ sind gleich oder verschieden, -O- -S-, -CO-, -CO-O- -O-CO-, - CO-S-, -S-CO-, -O-CO-O-, -$CH_2$-O-, -O-$CH_2$-, -$CH_2CH_2$-, -CH=CH- oder - C≡C-,

$M^4$ ist -$CH_2$-O-, -O-$CH_2$-, -CO-O-, -O-CO- oder eine Einfachbindung,

b, c, d, e, f, sind null oder eins, a und g sind eins,

* ist ein chirales Zentrum;

in ferroelektrischen Flüssigkristallmischungen.

**2.** Verwendung nach Anspruch 1 **dadurch gekennzeichnet, daß** die Verbindungen der Formel I der LC-Mischung in Konzentrationen von 0,01 bis 60 Gew. - % zugesetzt werden.

**3.** Verwendung nach Anspruch 1 oder 2, wobei die Symbole und Indices folgende Bedeutung haben:

$R^1$ ist ein geradkettiger oder verzweigter Alkylrest mit 1 bis 22 C-Atomen (mit oder ohne asymmetrisches C-Atom), wobei auch eine oder zwei nicht benachbarte -$CH_2$- Gruppen durch -O-, -S-, -CO-, -CO-O-, -O-CO-, -O-CO-O-, -CH=CH-, -C ≡C-, oder -$Si(CH_3)_2$- ersetzt sein können, oder ist eine der nachfolgenden chiralen Gruppen:

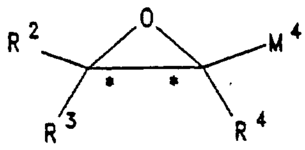 , 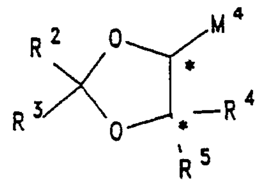 ,

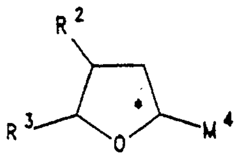 , 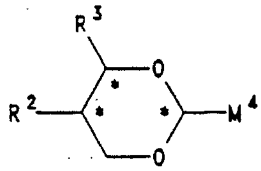 ,

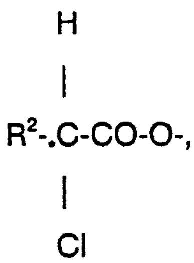 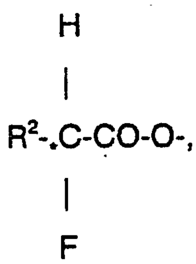 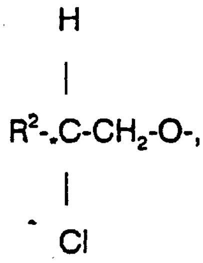 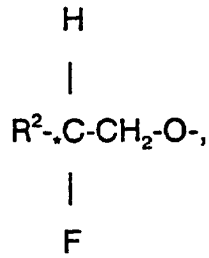

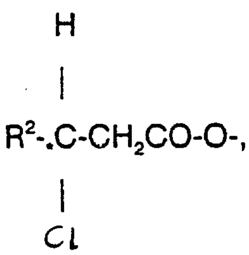 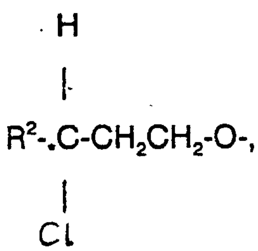

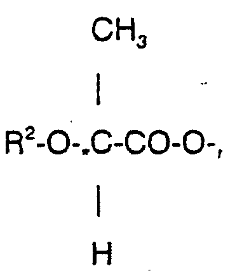 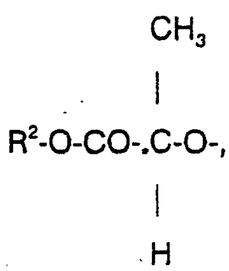

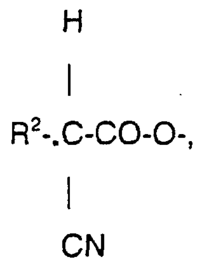 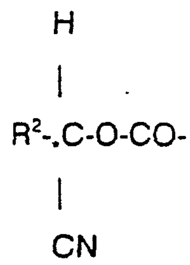

$R^2$, $R^3$, $R^4$, $R^5$ sind unabhängig voneinander H oder ein geradkettiger oder verzweigter Alkylrest mit 1 bis 22 C-Atomen, wobei auch eine oder zwei nicht benachbarte $-CH_2-$ Gruppen durch -O-, -S-, -CO-, -CO-O-, -O-CO-, -CO-S-, -S-CO-, -O-CO-O-, -CH=CH-, -C≡C-, oder $-Si(CH_3)_2-$ ersetzt sein können, oder $R^2$ und $R^3$ können zusammen auch $-(CH_2)_4-$ oder $-(CH_2)_5$ - sein, wenn sie als Substituenten an ein Dioxolan - System gebunden sind,

$A^1$, $A^2$, $A^3$, $A^4$ sind gleich oder verschieden 1,4-Phenylen, Pyrazin-2,5-diyl, Pyridazin-3,6-diyl, Pyridin - 2,5 - diyl, Pyrimidin - 2,5 - diyl, trans -1,4 - Cyclohexylen, (1,3,4) - Thiadiazol - 2,5 - diyl, 1,3 - Dioxan - 2,5 - diyl, Naphthalin - 2,6 - diyl oder Bicyclo[2.2.2]octan - 1,4 - diyl,

$M^1$, $M^2$, $M^3$ sind gleich oder verschieden -O-, -CO-, -CO-O-, -O-CO-, $-CH_2-O-$, $-O-CH_2-$, $-CH_2CH_2-$, -CH=CH- oder -C≡C-,

$M^4$ ist $-CH_2-O-$, $-O-CH_2-$, -CO-O-, -O-CO- oder eine Einfachbindung.

**4.** Verwendung nach Anspruch 1 oder 2, wobei die Symbole und Indices folgende Bedeutung haben:

$R^1$ ist ein geradkettiger oder verzweigter Alkylrest mit 1 bis 22 C-Atomen (mit oder ohne asymmetrisches C-Atom), wobei auch eine oder zwei nicht benachbarte-$CH_2$-Gruppen durch -O-, -CO-, -CO-O-, -O-CO-, -CH=CH-, -C≡C-, oder $-Si(CH_3)_2-$ ersetzt sein können, oder ist eine der nachfolgenden chiralen Gruppen:

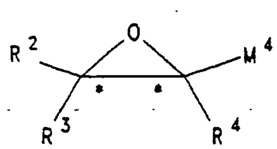 , 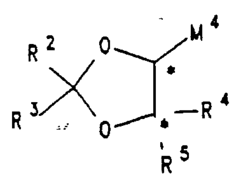 ,

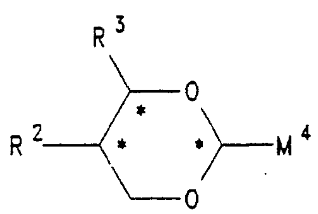 ,

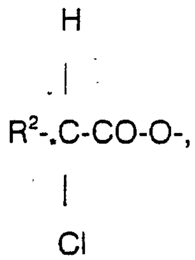
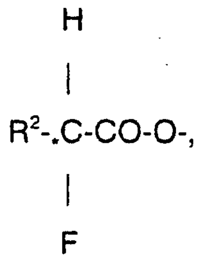
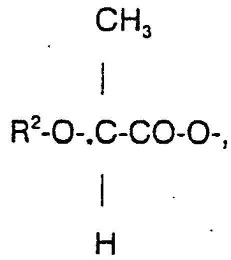
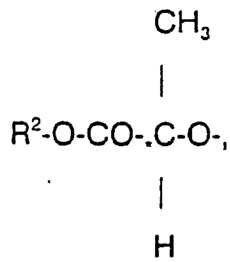

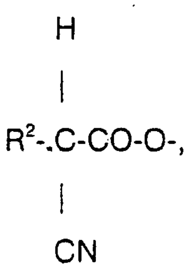

$R^2$, $R^3$, $R^4$, $R^5$ sind unabhängig voneinander H oder ein geradkettiger oder verzweigter Alkylrest mit 1 bis 22 C - Atomen, wobei auch eine oder zwei nicht benachbarte-$CH_2$- Gruppen durch -O-, -S-, -CO-, -CO-O, -O-CO-, -CO-S-, -S-CO-, -O-CO-O-, -CH=CH-, -C≡C- oder -$Si(CH_3)_2$- ersetzt sein können, oder $R^2$ und $R^3$ können zusammen auch -$(CH_2)_4$- oder -$(CH_2)_5$- sein, wenn sie als Substituenten an ein Dioxolan - System gebunden sind,

$A^1$, $A^2$, $A^3$, $A^4$ sind gleich oder verschieden 1,4-Phenylen, Pyrazin-2,5-diyl, Pyridin-2,5-diyl, Pyrimidin-2,5-diyl, trans-1,4-Cyclohexylen, (1,3,4)-Thiadiazol-2,5-diyl, Naphthalin-2,6-diyl oder 1,3 - Dioxan - 2,5 - diyl,

$M^1$, $M^2$, $M^3$ sind gleich oder verschieden, -O-, -CO-O-, -O-CO-, -$CH_2$-O-, -O-$CH_2$-, -$CH_2$-$CH_2$-, -CH=CH- oder -C≡C-,

$M^4$ ist -$CH_2$-O-, -O-$CH_2$-, -CO-O-, -O-CO- oder eine Einfachbindung.

**5.** Verwendung nach Anspruch 1 oder 2, **dadurch gekennzeichnet, daß** $R^1$ ein Alkylrest mit 1 bis 22 C-Atomen ist, wobei eine -$CH_2$- Gruppe durch -O-, -CH=CH- oder - $Si(CH_3)_2$ - ersetzt sein kann oder die chirale Gruppe

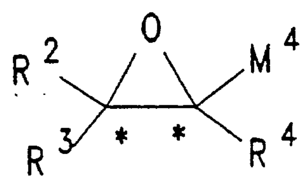

ist,
und die Gruppierung $(-A^1)_a(-M^1)_b-(-A^2)_c(-M^2)_d(-A^3)_e(-M^3)_f(-A^4)_g$ folgende Bedeutung hat:

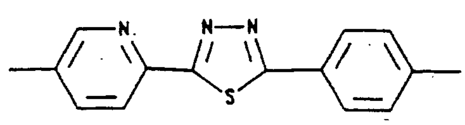

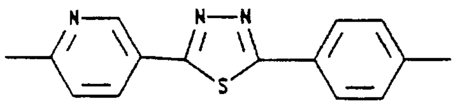 ,

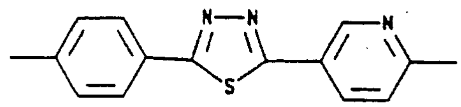 ,

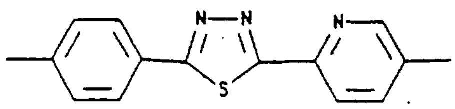 ,

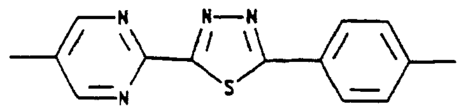 ,

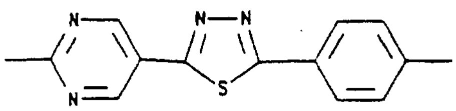 ,

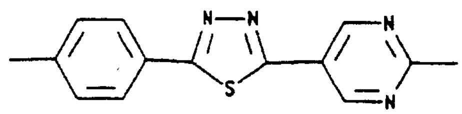 ,

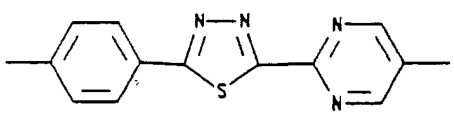 ,

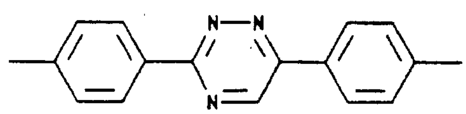 ,

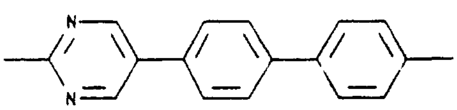 ,

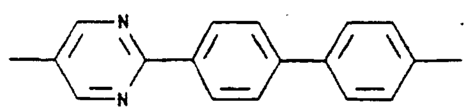 ,

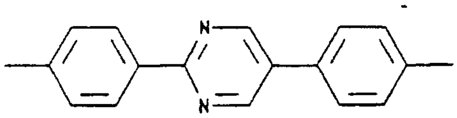 ,

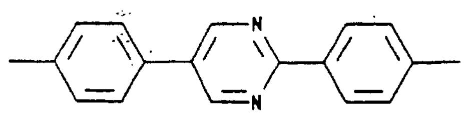 ,

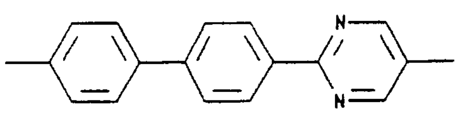 ,

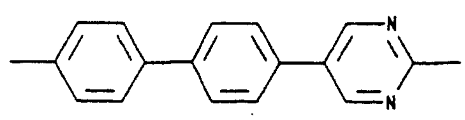 ,

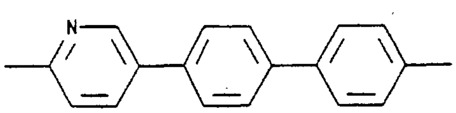 ,

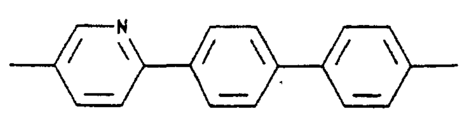 ,

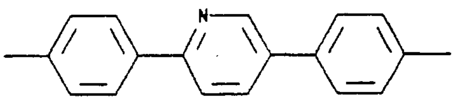 , 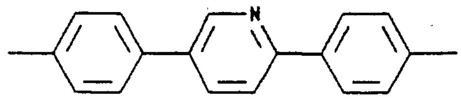 ,

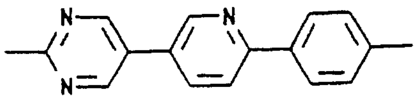 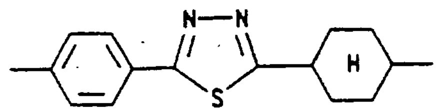

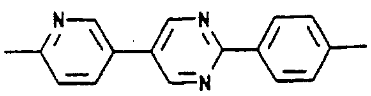 , 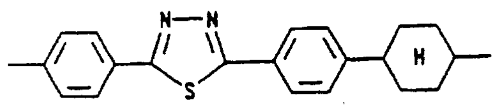

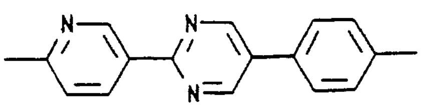 , 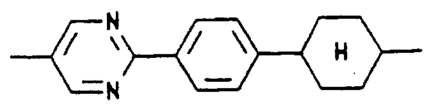

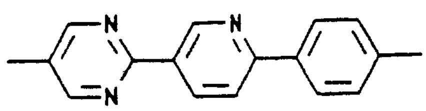

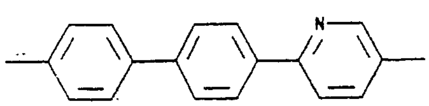 , 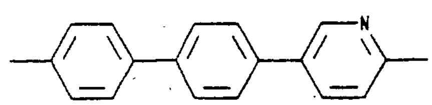 ,

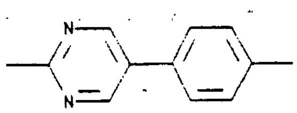 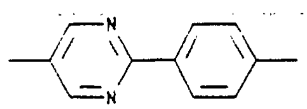 ,

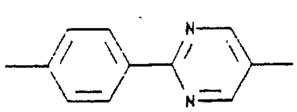 , 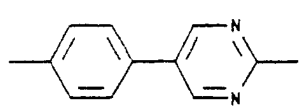 ,

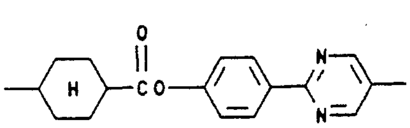 , 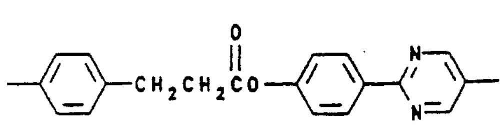

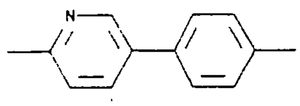 , 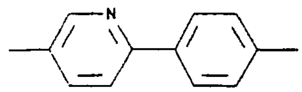 ,

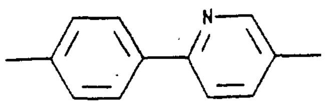 , 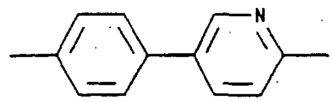 ,

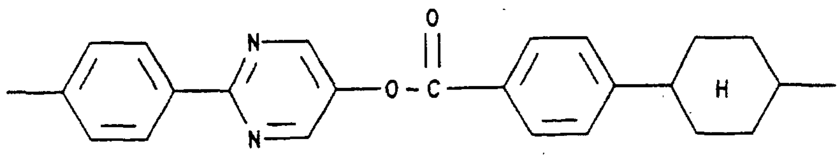 ,

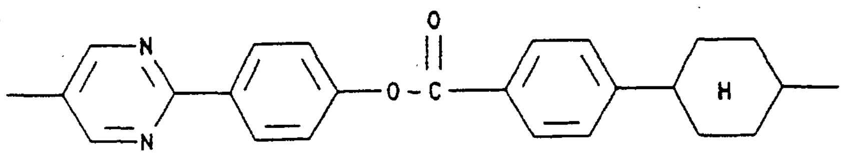 ,

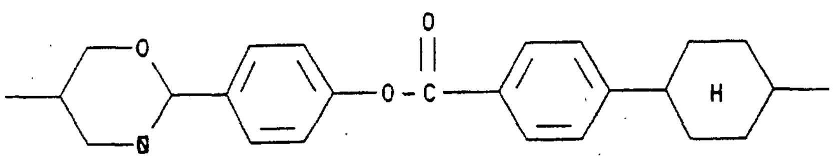 ,

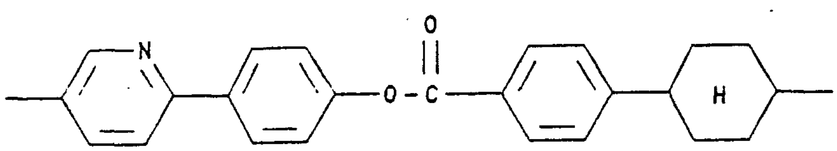 ,

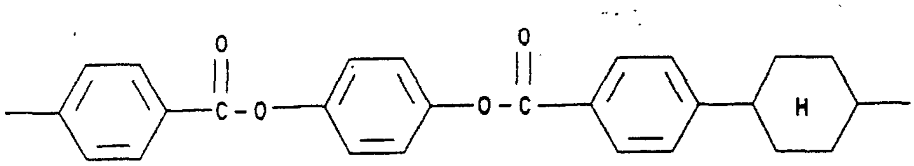

## Claims

**1.** The use of compounds of the formula I

$$R^1(-A^1)_a(-M^1)_b(-A^2)_c(-M^2)_d(-A^3)_e(-M^3)_f(-A^4)_g-H \quad (I)$$

in which the symbols and indices have the following meanings:

$R^1$ is a straight-chain or branched alkyl radical having 1 to 22 carbon atoms (with or without an asymmetrical carbon atom) in which, in addition, it is possible for one or two non-adjacent $-CH_2-$ groups to be replaced by -O-, -S-, -CO-, -CO-O-, -O-CO-, -CO-S-, -S-CO-, -O-CO-O-, -CH=CH-, -C≡C- or $-Si(CH_3)_2-$, and in which, in addition, one or more hydrogen atoms of the alkyl radical may be substituted by F, Cl, Br or CN, or is one of the chiral groups below:

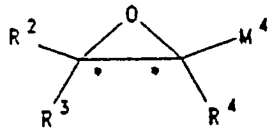 , 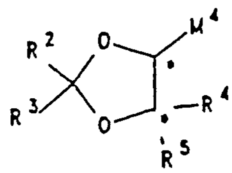 ,

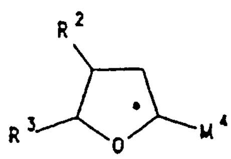 , 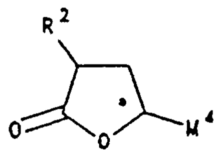 ,

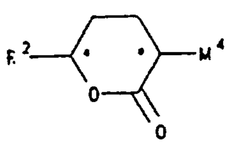 , 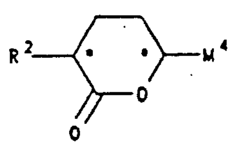 ,

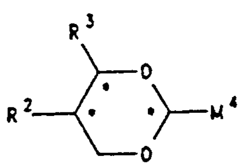 , 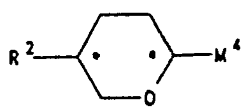 ,

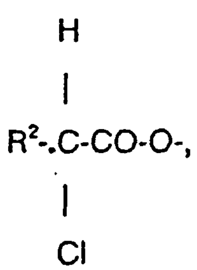 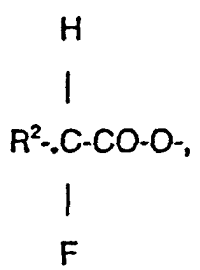 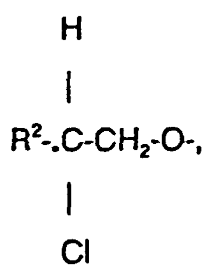 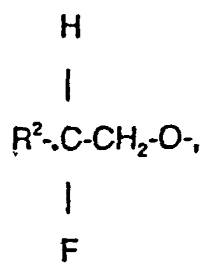

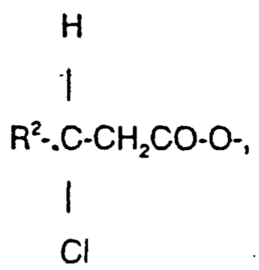 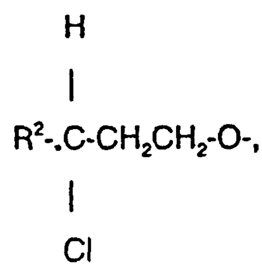

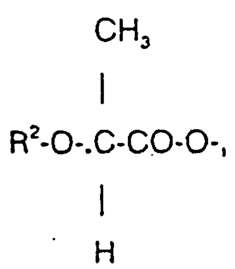 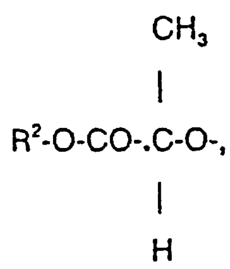

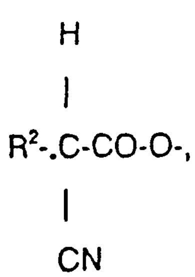 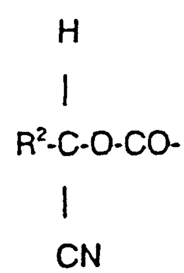

$R^2$, $R^3$, $R^4$ and $R^5$, independently of one another, are H or a straight-chain or branched alkyl radical having 1 to 22 carbon atoms in which, in addition, it is possible for one or two non-adjacent $-CH_2-$ groups to be replaced by -O-, -S-, -CO-, -CO-O-, -O-CO-, -CO-S-, -S-CO-, -O-CO-O-, -CH=CH-, -C≡C- or $-Si(CH_3)_2-$, or $R^2$ and $R^3$ together may alternatively be $-(CH_2)_4-$ or $-(CH_2)_5-$ if they are bonded as substituents to a dioxolane system;

$A^1$, $A^2$, $A^3$ and $A^4$ are identical or different and are 1,4-phenylene, in which one or two hydrogen atoms may be replaced by F, Cl and/or CN, pyrazine-2,5-diyl, pyridazine-3,6-diyl, pyridine-2,5-diyl, pyrimidine-2,5-diyl, in which one or two hydrogen atoms may be replaced by F, trans-1,4-cyclohexylene, 1,3,4-thiadiazole-2,5-diyl, 1,3-dioxane-2,5-diyl, 1,3-dithiane-2,5-diyl, 1,3-thiazole-2,4-diyl, 1,3-thiazole-2,5-diyl, thiophene-2,4-diyl, thiophene-2,5-diyl, piperazine-1,4-diyl,piperazine-2,5-diyl,naphthalene-2,6-diyl, bicyclo[2.2.2]octane-1,4-diyl, 1,3-dioxaborinane-2,5-diyl or trans-decalin-2,6-diyl;

$M^1$, $M^2$ and $M^3$ are identical or different and are -O-, -S-, -CO-, -CO-O-, -O-CO-, -CO-S-, -S-CO-, -O-CO-O-, $-CH_2-O-$, $-O-CH_2-$, $-CH_2CH_2-$, -CH=CH- or -C≡C-;

$M^4$ is $-CH_2-O-$, $-O-CH_2-$, -CO-O-, -O-CO- or a single bond;

b, c, d, e, f are zero or one, a and g are one;

* is a chiral center;

in ferroelectric liquid-crystal mixtures.

**2.** The use as claimed in claim 1, wherein the compounds of the formula I are added to the LC mixture in concentrations of from 0.01 to 60 % by weight.

**3.** The use as claimed in claim 1 or 2, in which the symbols and indices have the following meanings:

$R^1$ is a straight-chain or branched alkyl radical having 1 to 22 carbon atoms (with or without an asymmetrical carbon atom) in which, in addition, one or two non-adjacent $-CH_2-$ groups may be replaced by -O-, -S-, -CO-, -CO-O-, -O-CO-, -O-CO-O-, -CH=CH-, -C≡C- or $-Si(CH_3)_2-$, or is one of the chiral groups below:

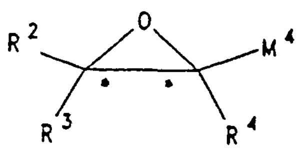 , 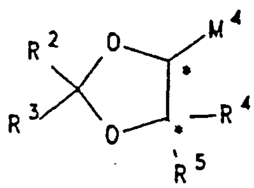 ,

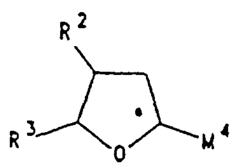 , 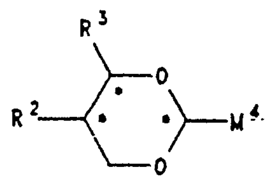 ,

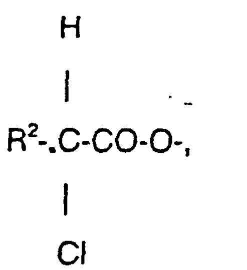 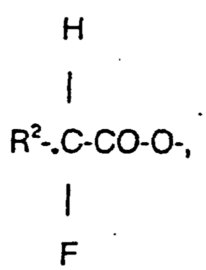 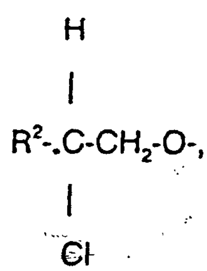 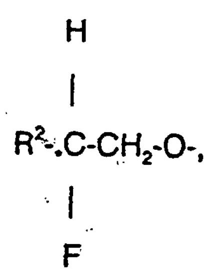

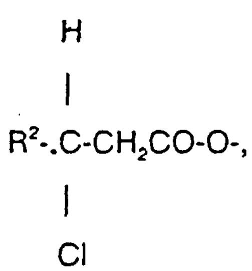 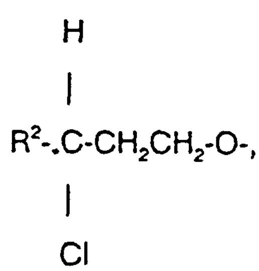

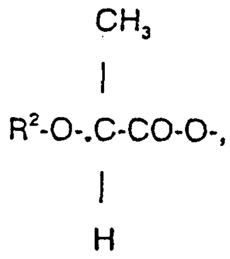

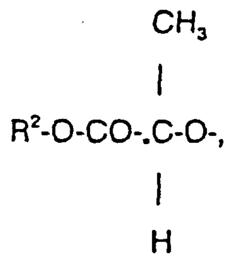

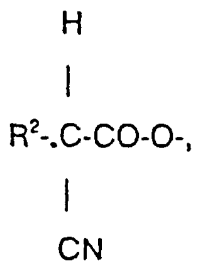

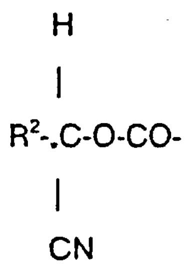

$R^2$, $R^3$, $R^4$ and $R^5$, independently of one another, are H or a straight-chain or branched alkyl radical having 1 to 22 carbon atoms in which, in addition, one or two non-adjacent $-CH_2-$ groups may be replaced by -O-, -S-, -CO-, -CO-O-, -O-CO-, -CO-S-, -S-CO-, -O-CO-O-, -CH=CH-, -C≡C- or $-Si(CH_3)_2-$, or $R^2$ and $R^3$ together may alternatively be $-(CH_2)_4-$ or $-(CH_2)_5-$ if they are bonded as substituents to a dioxolane system;

$A^1$, $A^2$, $A^3$ and $A^4$ are identical or different and are 1,4-phenylene, pyrazine-2,5-diyl, pyridazine-3,6-diyl, pyridine-2,5-diyl, pyrimidine-2,5-diyl, trans-1,4-cyclohexylene, 1,3,4-thidiazole-2,5-diyl, 1,3-dioxane-2,5-diyl, naphthalene-2,6-diyl or bicyclo[2.2.2]octane-1,4-diyl;

$M^1$, $M^2$ and $M^3$ are identical or different and are -O-, -CO-, -CO-O-, -O-CO-, $-CH_2-O-$, $-O-CH_2-$, $-CH_2CH_2-$, -CH=CH- or -C≡C-;

$M^4$ is $-CH_2-O-$, $-O-CH_2-$, -CO-O-, -0-CO- or a single bond.

**4.** The use as claimed in claim 1 or 2, in which the symbols and indices have the following meanings:

$R^1$ is a straight-chain or branched alkyl radical having 1 to 22 carbon atoms (with or without an asymmetrical carbon atom) in which, in addition, one or two non-adjacent $-CH_2-$ groups may be replaced by -O-, -CO-, -CO-O-, -O-CO-, -CH=CH-, -C≡C- or $-Si(CH_3)_2-$, or is one of the chiral groups below:

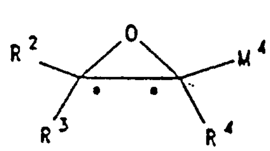
,
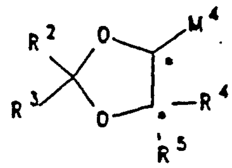
,

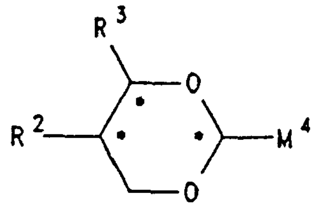

.

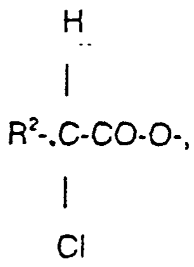

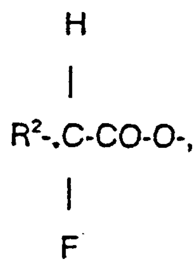

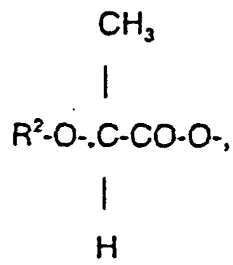

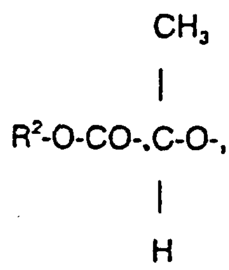

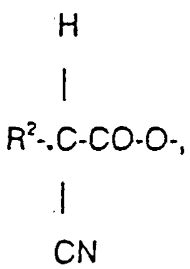

$R^2$, $R^3$, $R^4$ and $R^5$, independently of one another, are H or a straight-chain or branched alkyl radical having 1 to 22 carbon atoms in which, in addition, one or two non-adjacent $-CH_2-$ groups may be replaced by -O-, -S-, -CO-, -CO-O-, -O-CO-, -CO-S-, -S-CO-, -O-CO-O-, -CH=CH-, -C≡C- or $-Si(CH_3)_2-$, or $R^2$ and $R^3$ together may alternatively be $-(CH_2)_4-$ or $-(CH_2)_5-$ if they are bonded as substituents to a dioxolane system;

$A^1$, $A^2$, $A^3$ and $A^4$ are identical or different and are 1,4-phenylene, pyrazine-2,5-diyl, pyridine-2,5-diyl, pyrimidine-2,5-diyl, trans-1,4-cyclohexylene, 1,3,4-thiadiazole-2,5-diyl, naphthalene-2,6-diyl or 1,3-dioxane-2,5-diyl;

$M^1$, $M^2$ and $M^3$ are identical or different and are -O-, -CO-O-, -O-CO-, $-CH_2-O-$, $-O-CH_2-$, $-CH_2-CH_2-$, -CH=CH- or -C≡C-;

$M^4$ is $-CH_2-O-$, $-O-CH_2-$, -CO-O-, -O-CO- or a single bond.

**5.** The use as claimed in claim 1 or 2, wherein $R^1$ is an alkyl radical having 1 to 22 carbon atoms, in which one $-CH_2-$ group may be replaced by -O-, -CH=CH- or $-Si(CH_3)_2-$, or is the chiral group

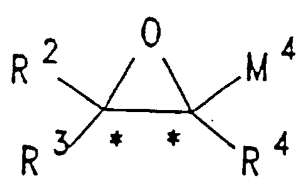

and the $(-A^1)_a(-M^1)_b-(-A^2)_c(-M^2)_d(-A^3)_e(-M^3)_f(-A^4)_g$ group has the following meaning:

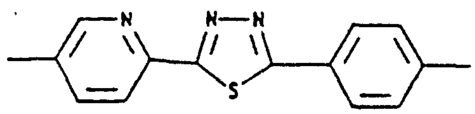,

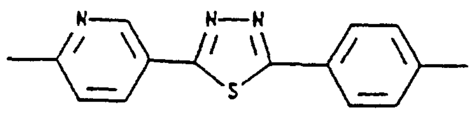, 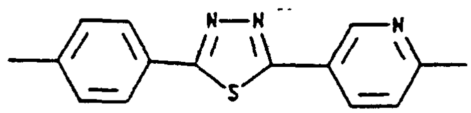,

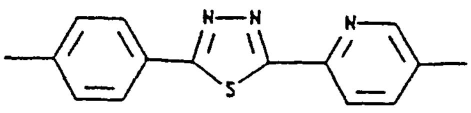, 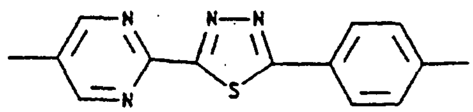,

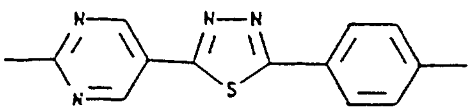, 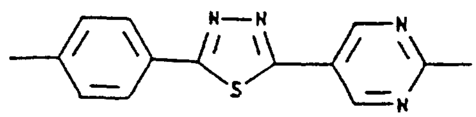,

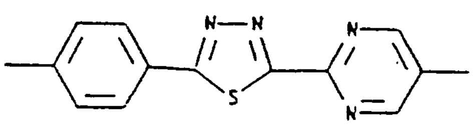, 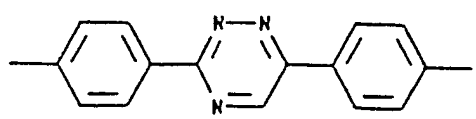,

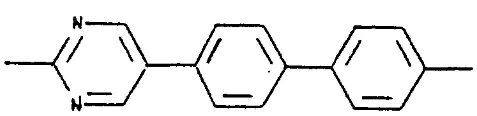, 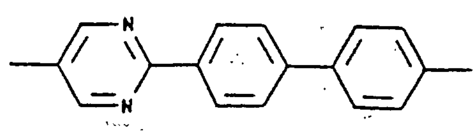,

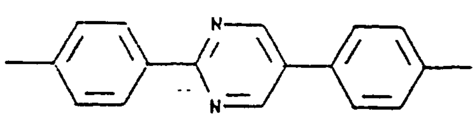, 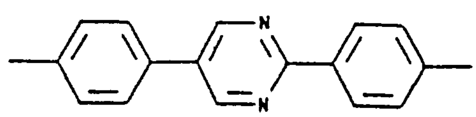,

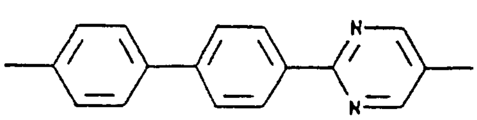, 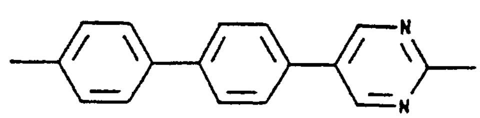.

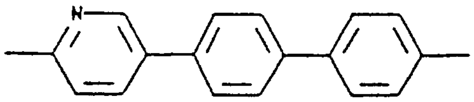 , 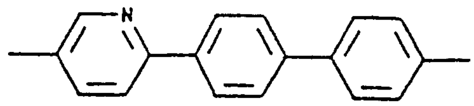 ,

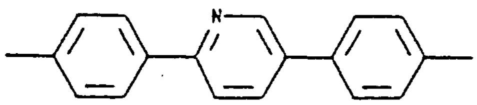 , 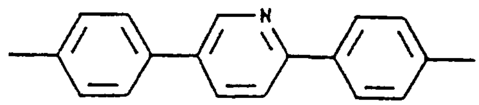 ,

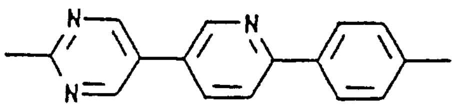 , 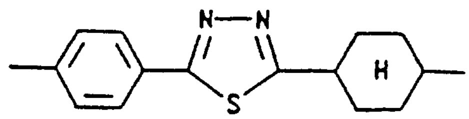

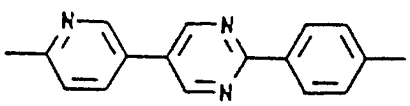 , 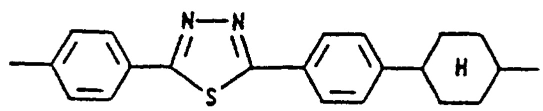

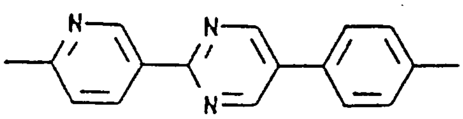 , 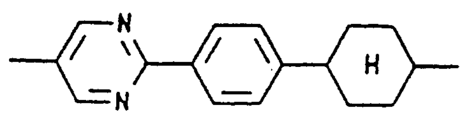

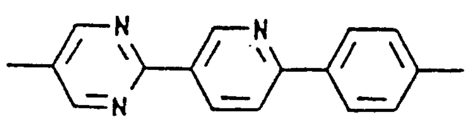

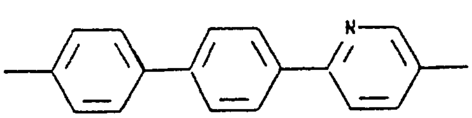 , 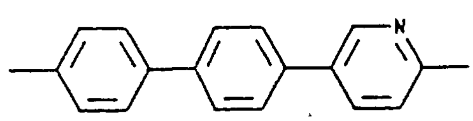 ,

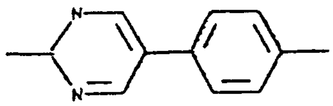 , 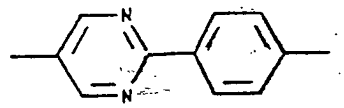 ,

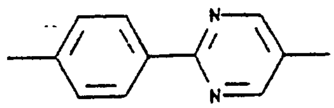 , 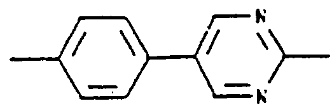 ,

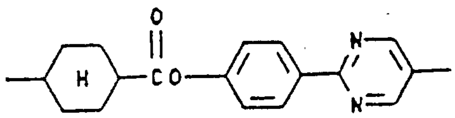 , 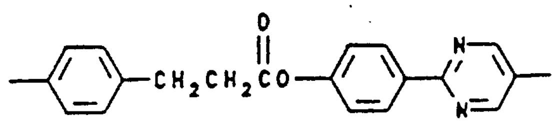

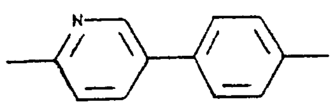 , 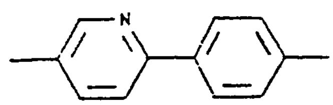 ,

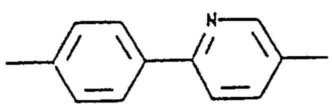 , 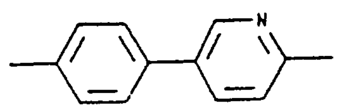 ,

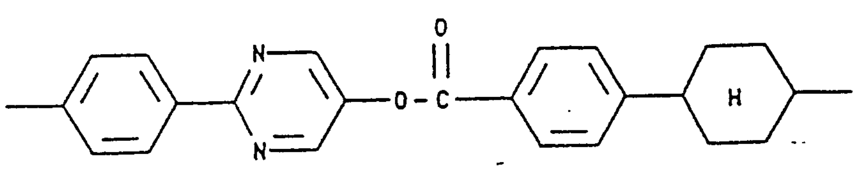 ,

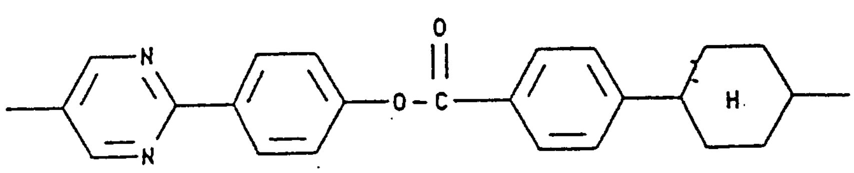 ,

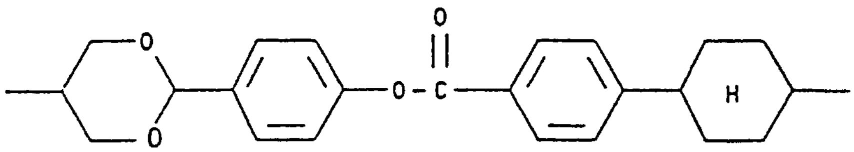

,

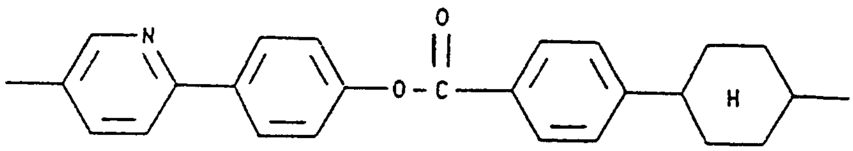

,

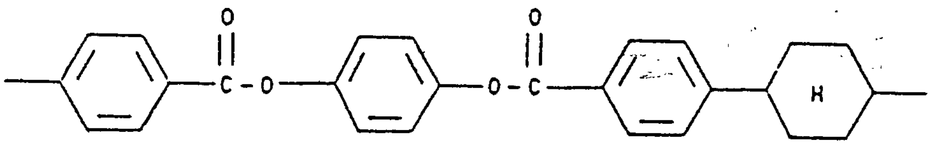

## Revendications

**1.** Utilisation de composés de formule générale I

$$R^1(-A^1)_a(-M^1)_b(-A^2)_c(-M^2)_d(-A^3)_e(-M^3)_r(-A^4)_g\text{-H} \quad (I)$$

dans laquelle les symboles et indices ont la signification suivante :

$R^1$ est un groupe alkyle à chaîne linéaire ou ramifiée ayant de 1 à 22 atomes de carbone (avec ou sans atome de carbone asymétrique), dans lequel également un ou deux groupes $-CH_2-$ non voisins peuvent être remplacés par -O-, -S-, -CO-, -CO-O-, -O-CO-, -CO-S-, -S-CO-, -O-CO-O-, -CH=CH-, -C≡C- ou $-Si(CH_3)_2-$ et dans lequel aussi un ou plusieurs atomes H du groupe alkyle peuvent être substitués par F, Cl, Br ou CN ou est un des groupes chiraux suivants :

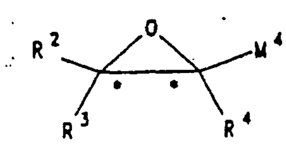 , 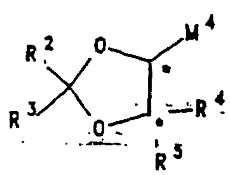 ,

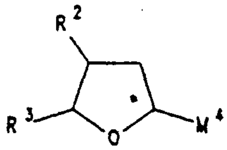 , 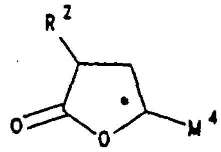 ,

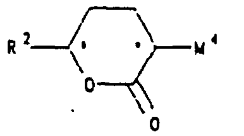,
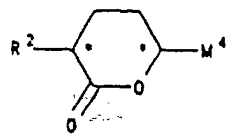,

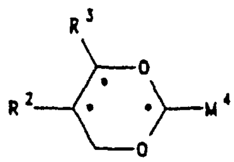,
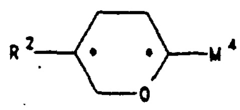,

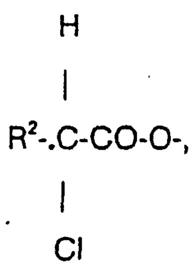
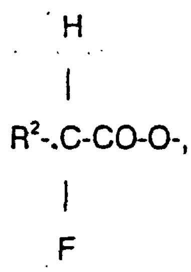
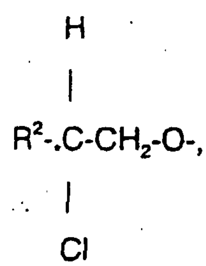
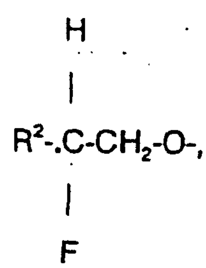

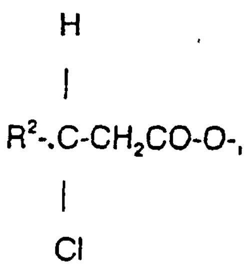
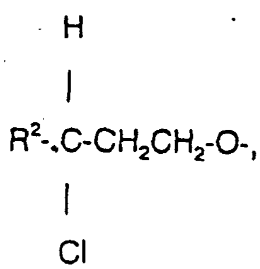

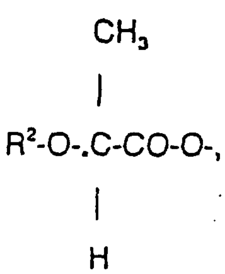
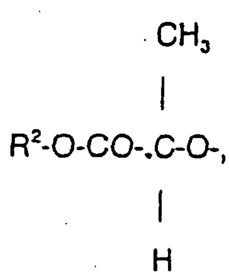

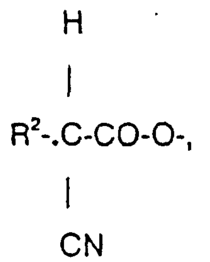 ,

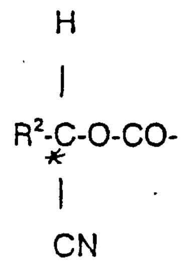

| | |
|---|---|
| $R^2$, $R^3$, $R^4$, $R^5$ | sont indépendamment les uns des autres H ou un groupe alkyle à chaîne linéaire ou ramifiée ayant de 1 à 22 atomes de carbone, dans lesquels aussi encore un ou deux groupes $-CH_2-$ non voisins peuvent être remplacés par -O-, -S-, -CO-, -CO-O-, -O-CO-, -CO-S-, -S-CO-, -O-CO-O-, -CH=CH-, -C≡C- ou $-Si(CH_3)_2-$, ou $R^2$ et $R^3$ peuvent ensemble aussi être $-(CH_2)_4-$ ou $-(CH_2)_5-$, lorsqu'ils sont liés comme substituants à un système dioxolane, |
| $A^1$, $A^2$, $A^3$ et $A^4$ | sont, identiques ou différents, 1,4-phénylène, dans lequel un ou deux atomes H peuvent être remplacés par F, Cl et/ou CN, pyrazine-2,5-diyle, pyridazine-3,6-diyle, pyridine-2,5-diyle, pyrimidine-2,5-diyle, dans lequel ou deux atomes H peuvent être remplacés par F, trans-1,4-cyclohexylène, (1,3,4)-thiadiazol-2,5-diyle, 1,3-dioxane-2,5-diyle, 1,3-dithiane-2,5-diyle, 1,3-thiazol-2,4-diyle, 1,3-thiazol-2,5-diyle, thiophène-2,4-diyle, thiophène-2,5-diyle, pipérazine-1,4-diyle, pipérazine-2,5-diyle, naphtalène-2,6-diyle, bicyclo[2.2.2]octane-1,4-diyle, 1,3-dioxaborinane-2,5-diyle ou trans-décaline-2,6-diyle, |
| $M^1$, $M^2$, $M^3$ | sont, identiques ou différents, -O-, -S-, -CO-, -CO-O-, -O-CO-, -CO-S-, -S-CO-, -O-CO-O-, $-CH_2-O-$, $-O-CH_2-$, $-CH_2CH_2-$, -CH=CH- ou -C≡C-, |
| $M^4$ | est $-CH_2-O-$, $-O-CH_2-$, -CO-O-, -O-CO- ou une liaison simple, |
| b, c, d, e, f | sont zéro ou un et g, a sont un, |
| * | est un centre chiral ; |

dans des mélanges de cristaux liquides ferroélectriques.

**2.** Utilisation selon la revendication 1, **caractérisée en ce que** les composés de formule I sont ajoutés au mélange LC en concentrations de 0,01 à 60 % en poids.

**3.** Utilisation selon la revendication 1 ou 2, dans laquelle les symboles et indices ont la signification suivante :

| | |
|---|---|
| $R^1$ | est un groupe alkyle à chaîne linéaire ou ramifiée ayant de 1 à 22 atomes de carbone (avec ou sans atome de carbone asymétrique), dans lequel également un ou deux groupes $-CH_2-$ non voisins peuvent être remplacés par -O-, -S-, -CO-, -CO-O-, -O-CO-, -O-CO-O-, -CH=CH-, -C≡C- ou $-Si(CH_3)_2-$ ou est un des groupes chiraux suivants : |

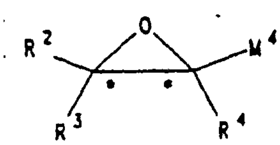 ,

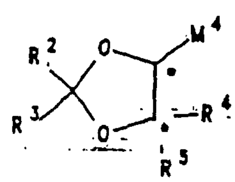 ,

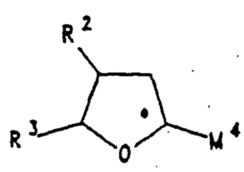 ,

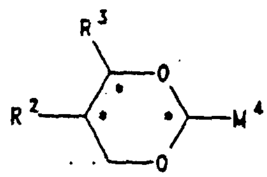 ,

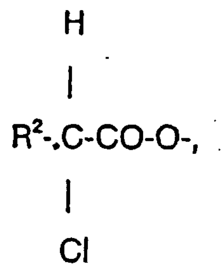

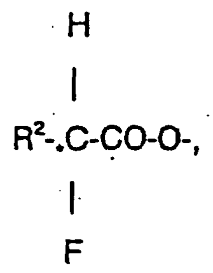

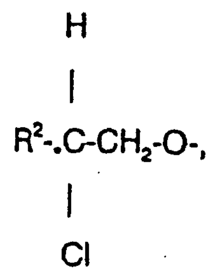

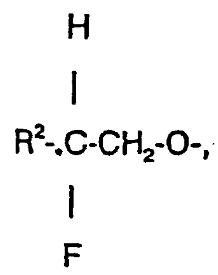

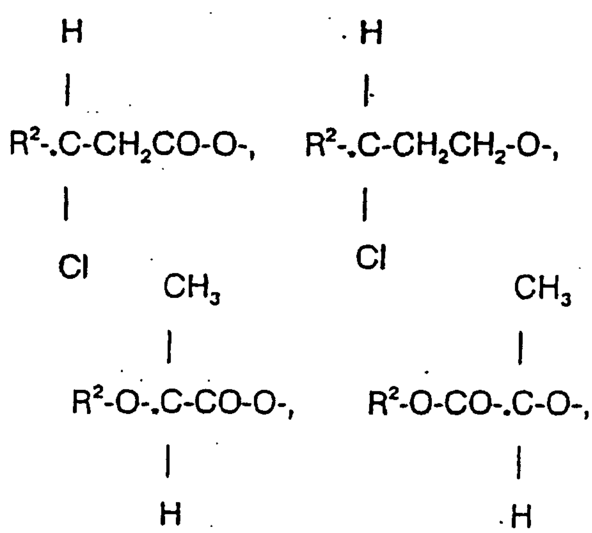

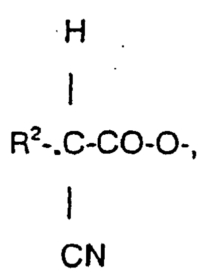

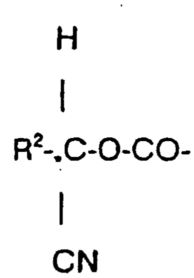

| | |
|---|---|
| $R^2$, $R^3$, $R^4$, $R^5$ | sont indépendamment les uns des autres H ou un groupe alkyle à chaîne linéaire ou ramifiée ayant de 1 à 22 atomes de carbone, dans lesquels aussi encore un ou deux groupes $-CH_2-$ non voisins peuvent être remplacés par -O-, -S-, -CO-, -CO-O-, -O-CO-, -CO-S-, -S-CO-, -O-CO-O-, -CH=CH-, -C≡C- ou $-Si(CH_3)_2-$, ou $R^2$ et $R^3$ peuvent ensemble aussi être $-(CH_2)_4-$ ou $-(CH_2)_5-$, lorsqu'ils sont liés comme substituants à un système dioxolane, |
| $A^1$, $A^2$, $A^3$ et $A^4$ | sont, identiques ou différents, 1,4-phénylène, pyrazine-2,5-diyle, pyridazine-3,6-diyle, pyridine-2,5-diyle, pyrimidine-2,5-diyle, trans-1,4-cyclohexylène, (1,3,4)-thiadiazol-2,5-diyle, 1,3-dioxane-2,5-diyle, naphtalène-2,6-diyle ou bicyclo[2.2.2]octane-1,4-diyle, |
| $M^1$, $M^2$, $M^3$ | sont, identiques ou différents, -O-, -CO-, -CO-O-, -O-CO-, $-CH_2-O-$, $-O-CH_2-$, $-CH_2CH_2-$, -CH=CH- ou -C≡C-, |
| $M^4$ | est $-CH_2-O-$, $-O-CH_2-$, -CO-O-, -O-CO- ou une liaison simple. |

**4.** Utilisation selon la revendication 1 ou 2 dans laquelle les symboles et indices ont la signification suivante :

$R^1$ est un groupe alkyle à chaîne linéaire ou ramifiée ayant de 1 à 22 atomes de carbone (avec ou sans atome de carbone asymétrique), dans lequel également un ou deux groupes $-CH_2-$ non voisins peuvent être remplacés par -O-, -CO-, -CO-O-, -0-CO-, -CH=CH-, -C≡C- ou $-Si(CH_3)_2$, ou est un des groupes chiraux suivants :

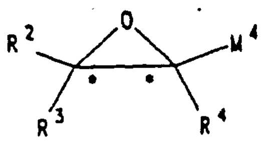
,
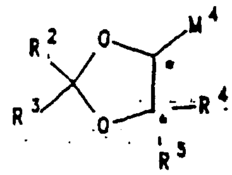
,
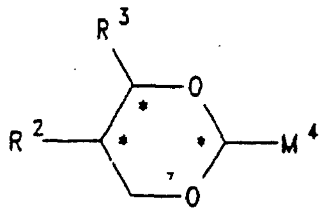
,

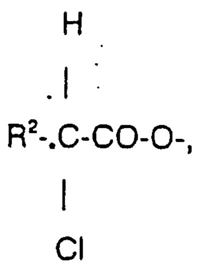
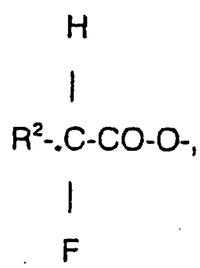
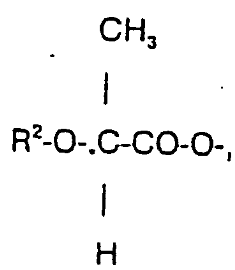
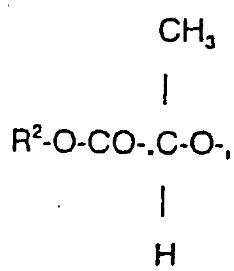

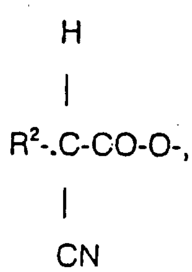

$R^2$, $R^3$, $R^4$, $R^5$ sont indépendamment les uns des autres H ou un groupe alkyle à chaîne linéaire ou ramifiée ayant de 1 à 22 atomes de carbone, dans lesquels aussi encore un ou deux groupes $-CH_2-$ non voisins peuvent être remplacés par -O-, -S-, -CO-, -CO-O-, -O-CO-, -CO-S-, -S-CO-, -O-CO-O-, -CH=CH-, -C≡C- ou $-Si(CH_3)_2$, ou $R^2$ et $R^3$ peuvent ensemble aussi être $-(CH_2)_4-$ ou $-(CH_2)_5-$, lorsqu'ils sont liés comme substituants à un système dioxolane,

$A^1$, $A^2$, $A^3$ et $A^4$ sont, identiques ou différents, 1,4-phénylène, pyrazine-2,5-diyle, pyridine-2,5-diyle, pyrimidine-2,5-diyle, trans-1,4-cyclohexylène, (1,3,4)-thiadiazol-2,5-diyle, naphtalène-2,6-diyle ou 1,3-dioxane-2,5-diyle,

$M^1$, $M^2$, $M^3$ sont, identiques ou différents, -O-, -CO-O-, -O-CO-, $-CH_2-O-$, $-O-CH_2-$, $-CH_2-CH_2-$, -CH=CH- ou -C≡C-,

$M^4$ est $-CH_2-O-$, $-O-CH_2-$, -CO-O-, -O-CO- ou une liaison simple.

**5.** Utilisation selon la revendication 1 ou 2, **caractérisée en ce que** $R^1$ est un groupe alkyle ayant de 1 à 22 atomes de carbone, dans lequel un groupe $-CH_2-$ peut être remplacé par -O-, -CH=CH- ou $-Si(CH_3)_2-$ ou est le groupe chiral

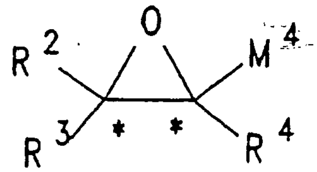

et le groupe $(-A^1)_a(-M^1)_b(-A^2)_c(-M^2)_d(-A^3)_e(-M^3)_r(-A^4)_g$ a la signification suivante :

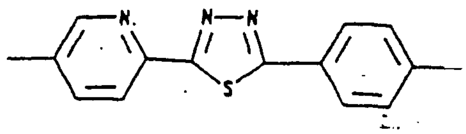

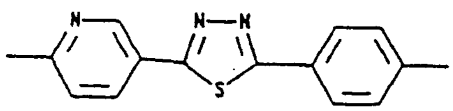,
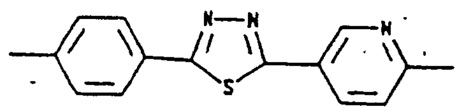,

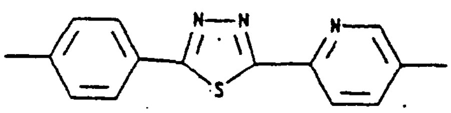,
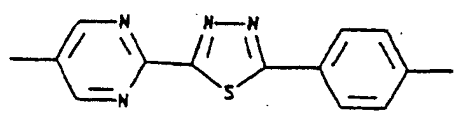,

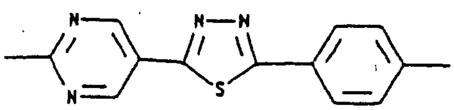,
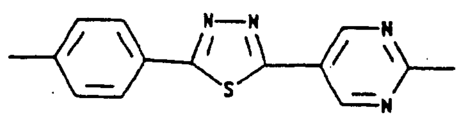,

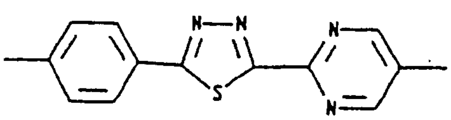,
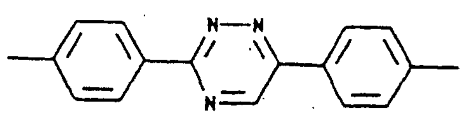,

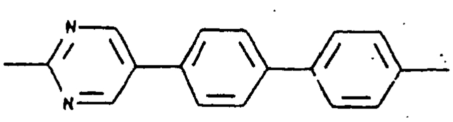,
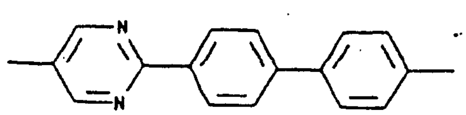.

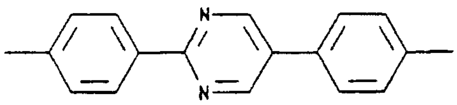 , 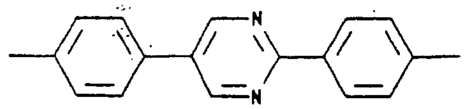 ,

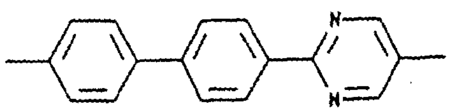 , 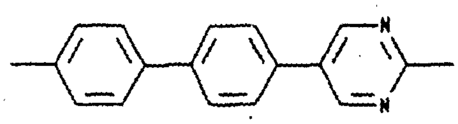 ,

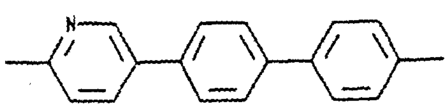 , 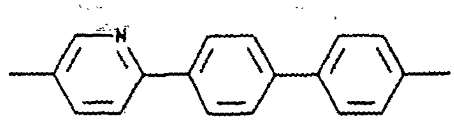 ,

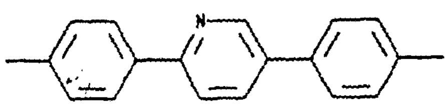 , 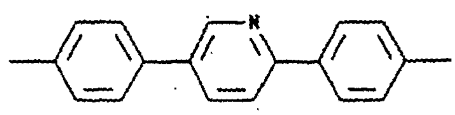 ,

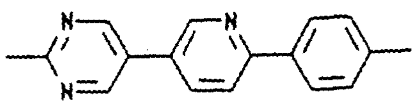 , 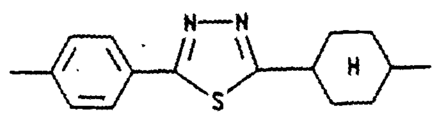

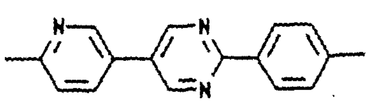 , 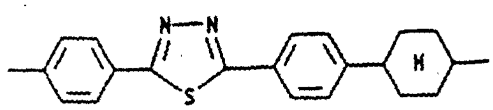

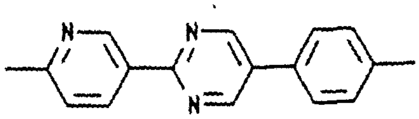 , 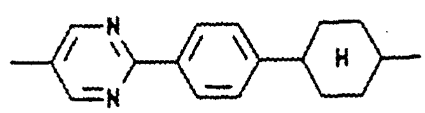

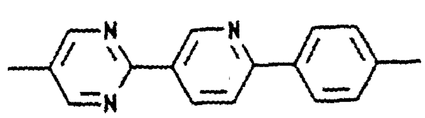

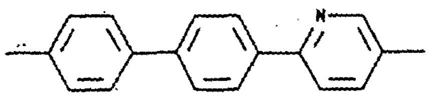 , 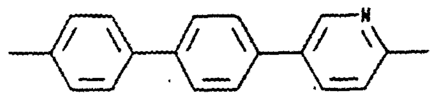 ,

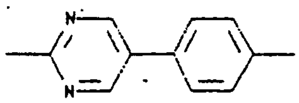 , 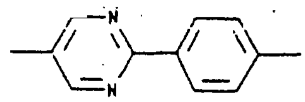 ,

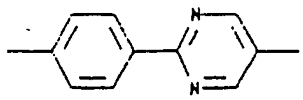 , 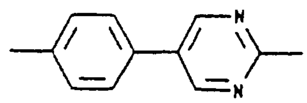 ,

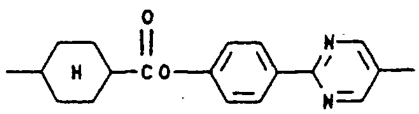 , 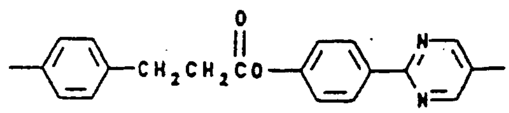

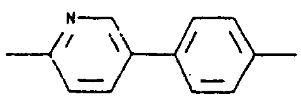 , 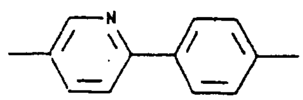 ,

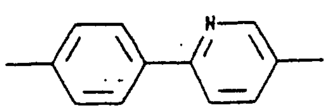 , 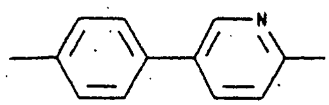 ,

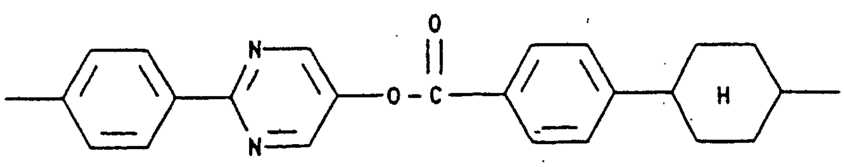 ,

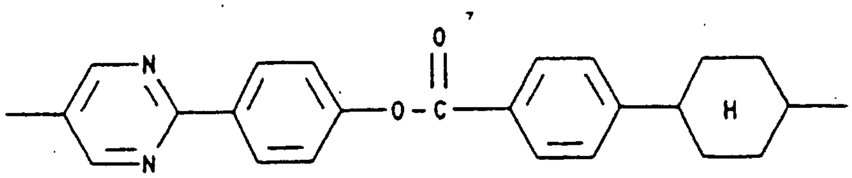 ,

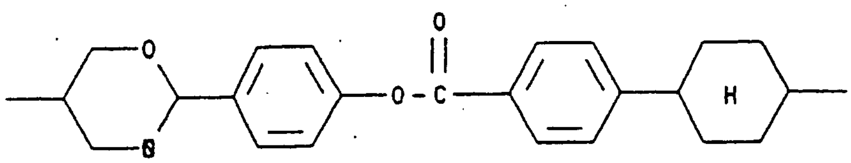

,

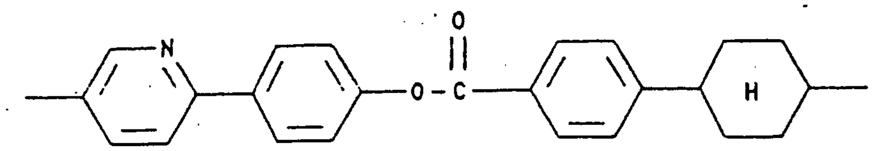

,